# EUROPEAN PATENT APPLICATION

(11) **EP 4 847 519 A1**
(43) Date of publication of application: **02.12.2026**
(21) Application number: 25744688.0
(22) Date of filing: 23.01.2025
(51) Int. Cl.: C07J 17/00, C07J 9/00, A61K 31/57, A61K 31/58, A61P 37/06, A61P 35/02, A61P 35/00, A61P 17/10

(54) **HIGH-EFFICACY GLUCOCORTICOID COMPOUND, AND PREPARATION AND USE THEREOF**

(30) Priority: 24.01.2024 CN 202410102688
(71) Applicant: Zhejiang Palo Alto Pharmaceuticals, Inc., Quzhou, Zhejiang 324099 (CN)
(72) Inventor: XI, Zhijian, Quzhou, Zhejiang 324099 (CN); CAO, Jianxin, Quzhou, Zhejiang 324099 (CN); LU, Chunping, Quzhou, Zhejiang 324099 (CN); YU, Hua, Quzhou, Zhejiang 324099 (CN)
(74) Representative: JD&P Patent Attorneys Joanna Dargiewicz & Partners
(86) International application number: PCT/CN2025/074375
(87) International publication number: WO 2025/157225

(57) **Abstract**

The present invention provides a high potency glucocorticoid compound, a method of preparation and use thereof. Specifically, the present invention provides a high potency glucocorticoid compound represented by Formula I, a method of preparation and use thereof. The glucocorticoid compound of the present invention can achieve the same effect at a lower dose, thereby minimizing side effects.

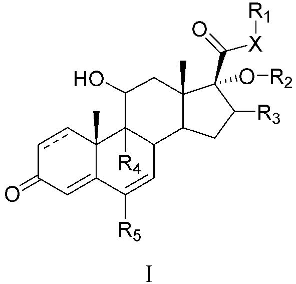

## Description

### TECHNICAL FIELD

The present invention relates to the field of medicine, in particular to a high potency glucocorticoid compound, a method of preparation and use thereof, and use of a composition containing the high potency glucocorticoid compound.

### BACKGROUND

Glucocorticoids such as prednisone, dexamethasone (DEX), and budesonide are highly effective anti-inflammatory drugs. They are widely used for treating inflammatory and autoimmune diseases, such as rhinitis, asthma, chronic obstructive pulmonary disease, dermatitis, arthritis, lupus, and Crohn's disease. These drugs exert their physiological effects by binding to the glucocorticoid receptor (GR), a ligand-activated transcription factor of the nuclear receptor superfamily. In the absence of glucocorticoids, GR resides in the cytoplasm and is bound to chaperone proteins such as hsp90 and hsp70. Binding of the hormone induces a conformational change in GR, resulting in its translocation to the nucleus, where it exerts its transcriptional control activities, namely activation (transactivation) or inhibition (transrepression). In transactivation, GR dimerizes, directly binds to specific glucocorticoid response elements, and then recruits co-activators to activate transcription. In transrepression, the general model is as follows: GR binds to other transcription factors (e.g., NF-κB, AP-1) to indirectly tether to their binding sites through protein-protein interactions. When tethered near the target promoter, GR inhibits downstream gene expression. It is generally believed that transrepression does not require GR dimerization.

Transrepression is the primary mechanism by which glucocorticoids act as anti-inflammatory drugs. Tethering of GR to NF-κB/AP-1 promoters leads to transcriptional inhibition of major downstream pro-inflammatory factors, including pro-inflammatory cytokines (e.g., TNF-α, IL-1, and IL-6), chemokines (e.g., CCL2, CCL19), and enzymes associated with the onset of inflammation (e.g., COX2, MMP13, and phospholipase A2). Due to the rapid action and sustained effects of glucocorticoids, they remain the treatment of choice for inflammatory diseases. However, long-term use of glucocorticoids, especially at high doses, has many adverse consequences, including diabetes/glucose intolerance, hypertension, obesity, and osteoporosis. Most of these consequences are attributed to the transactivation of GR. For example, glucocorticoids induce genes encoding the rate-limiting enzymes of the gluconeogenesis pathway in the liver, glucose-6-phosphatase and phosphoenolpyruvate carboxykinase, thereby increasing de novo synthesis of glucose and ultimately leading to weight gain or diabetes. Glucocorticoids also induce Dickkopf-1 (DKK1), a key regulatory gene for bone development, whose upregulation leads to osteoporosis and bone loss. Many of the side effects of glucocorticoids are commonly observed to be associated with high-dose glucocorticoid use (Hoes JN, et al. (2009) Adverse events of low-to medium-dose oral glucocorticoids in inflammatory diseases: a meta-analysis. Ann Rheum Dis 68 (12): 1833-1838; Spies CM, et al. (2011) Glucocorticoids. Best Pract Res Cl Rh 25(6): 891-900; Hoes JN, Jacobs JW, Buttgereit F, Bijlsma JW (2010) Current view of glucocorticoid co-therapy with DMARDs in rheumatoid arthritis. Nat Rev Rheumatol 6 (12): 693-702.). For example, a "threshold pattern" is observed with the use of prednisone: at 7.5 mg per day, it can cause glaucoma, depression, and hypertension. These side effects are caused by GR transactivation as well as off-target activation of other receptors, such as the mineralocorticoid receptor (MR), whose activation can cause hypertension (Frey FJ, Odermatt A, Frey B M (2004) Glucocorticoid-mediated mineralocorticoid receptor activation and hypertension. Curr Opin Nephrol Hypertens 13 (4): 451-458.). Therefore, it is important to develop high-potency and selective glucocorticoids to reduce unwanted side effects.

Potency and efficacy are two key pharmacokinetic parameters of glucocorticoids. While efficacy is the maximum activity achievable for a given drug, usually at maximal concentrations, potency is the concentration of a given drug required to achieve half-maximal activity (EC₅₀). For two glucocorticoids with the same efficacy, a high potency glucocorticoid will require a lower dose to achieve the same therapeutic effect (Hoes JN, Jacobs JW, Buttgereit F, Bijlsma JW (2010) Current view of glucocorticoid co-therapy with DMARDs in rheumatoid arthritis. Nat Rev Rheumatol 6 (12): 693-702; Frey FJ, Odermatt A, Frey B M (2004) Glucocorticoid-mediated mineralocorticoid receptor activation and hypertension. Curr Opin Nephrol Hypertens 13 (4): 451-458.). Importantly, glucocorticoids may have different potencies for transactivation and transrepression; for example, gene induction by GR via DEX requires a 5- to 6-fold higher glucocorticoid concentration than gene suppression. This differential response provides an opportunity to develop high potency glucocorticoids that can be used at low doses to achieve complete inhibition of inflammatory signals with minimal transactivation activity and side effects. Finally, the development of insensitivity and resistance to glucocorticoid therapy is a major problem in treating common inflammatory diseases such as chronic obstructive pulmonary disease, rheumatoid arthritis, and inflammatory bowel disease. Glucocorticoid resistance is also an unsolved problem for leukocyte cancers, especially childhood acute leukemia. Several mechanisms of glucocorticoid resistance have been identified or proposed, including alterations in kinase pathways, changes in cofactors, and deletion or mutation of the receptor. A common observation is a reduced affinity of the ligand for the receptor in glucocorticoid-resistant patients. Such patients treated with high potency glucocorticoids have shown improvement, but the effect gradually diminishes (Gaynon PS, Carrel AL (1999) Glucocorticosteroid therapy in childhood acute lymphoblastic leukemia. Adv Exp Med Biol 457:593-605).

Therefore, there is an urgent need to develop a new generation of more potent glucocorticoids.

### SUMMARY

To solve the above problems, the present application provides a high potency glucocorticoid compound, and a method of preparation and use thereof. The glucocorticoid compound of the present invention can achieve the same effect at a lower dose, thereby minimizing side effects.

In a first aspect of the present invention, a compound represented by Formula I, or a pharmaceutically acceptable salt thereof, is provided, 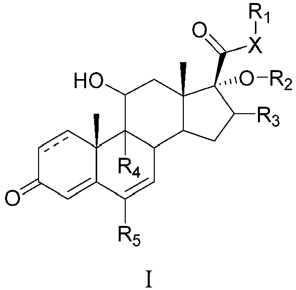 wherein, X is O or S;
-̅ -̅ -̅ is a single bond or a double bond;
R₁ is selected from the group consisting of hydrogen, C₁₋₆ alkyl, C₃₋₁₀ cycloalkyl, C₆₋₁₂ aryl, 5- to 15-membered heteroaryl, C₅₋₁₅ aralkyl, heteroaralkyl, and 3- to 10-membered heterocycloalkyl, wherein 0, 1, 2 or 3 hydrogen atoms in each of the foregoing groups are substituted with Rₐ;
R₂ is -L-R', wherein L is a bond or -C(O)-, and R' is selected from the group consisting of hydrogen, C₁₋₆ alkyl, C₃₋₁₀ cycloalkyl, C₆₋₁₂ aryl, 5- to 15-membered heteroaryl, C₅₋₁₅ aralkyl, heteroaralkyl, and 3- to 10-membered heterocycloalkyl, wherein 0, 1, 2 or 3 hydrogen atoms in the foregoing groups are substituted with R_{b};
R₃ is selected from the group consisting of hydrogen and C₁₋₆ alkyl, wherein 0, 1, 2 or 3 hydrogen atoms in the C₁₋₆ alkyl are substituted with R_{c};
R₄ and R₅ are each independently selected from the group consisting of hydrogen, halogen, and C₁₋₄ alkyl; wherein 0, 1, 2 or 3 hydrogen atoms in the C₁₋₄ alkyl are substituted with R_{d};
Rₐ, R_{b}, R_{c} and R_{d} are each independently selected from the group consisting of hydroxyl, halogen, CN, oxo, carboxyl, amino, sulfonic acid group, C₁₋₆ alkyl, and C₁₋₆ alkoxy.

In a preferred embodiment, R₁ is selected from the group consisting of hydrogen and C₁₋₆ alkyl, wherein 0, 1, 2 or 3 hydrogen atoms in the foregoing C₁₋₆ alkyl are substituted with Rc;
preferably, R₁ is selected from the group consisting of methyl, ethyl, propyl, isopropyl, butyl, isobutyl, and tert-butyl, wherein 0, 1, 2 or 3 hydrogen atoms in the foregoing groups are substituted with halogen;
more preferably, R₁ is fluoromethyl.

In a preferred embodiment, R₁ is selected from the group consisting of C₃₋₇ cycloalkyl, C₆₋₁₀ aryl, phenyl, and naphthyl, wherein 1, 2 or 3 hydrogen atoms in each of the foregoing groups are substituted with Rₐ.

In a preferred embodiment, R₂ is -C(O)-R', and R' is selected from the group consisting of hydrogen, C₁₋₆ alkyl, C₆₋₁₀ aryl(C₁₋₆ alkyl), 5- to 15-membered heteroaryl, and 5- to 15-membered heteroaryl(C₁₋₆ alkyl), wherein 0, 1, 2 or 3 hydrogen atoms in the foregoing groups are substituted with R_{b}, and R_{b} is selected from the group consisting of halogen, CN, carboxyl, and oxo;
preferably, R₂ is selected from the group consisting of

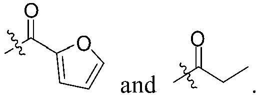

In a preferred embodiment, R₃ is selected from the group consisting of methyl, ethyl, propyl, isopropyl, butyl, isobutyl, and tert-butyl, wherein 1, 2 or 3 hydrogen atoms in the foregoing groups are substituted with R_{c}.

In a preferred embodiment, R₄ and R₅ are each independently -F.

In a preferred embodiment, it is characterized in that, the compound of Formula I is selected from the group consisting of:

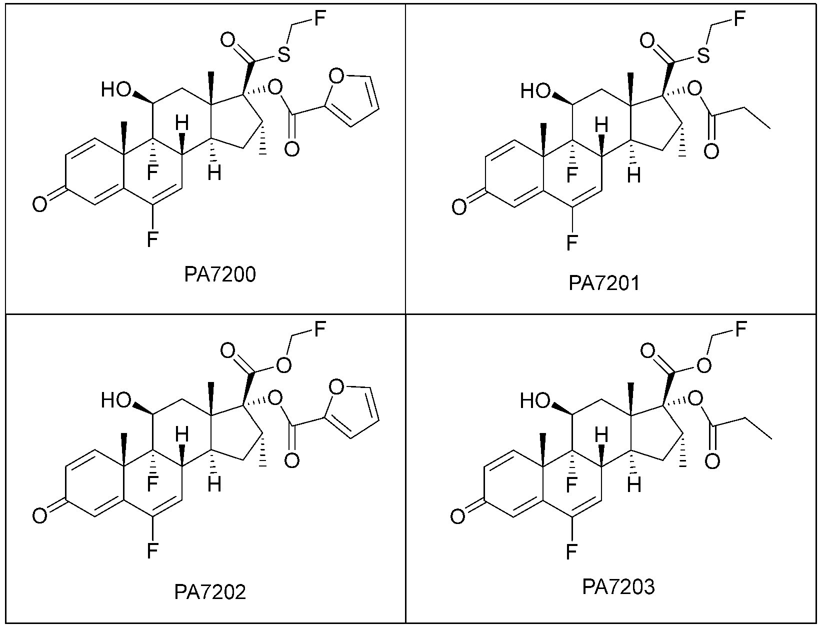

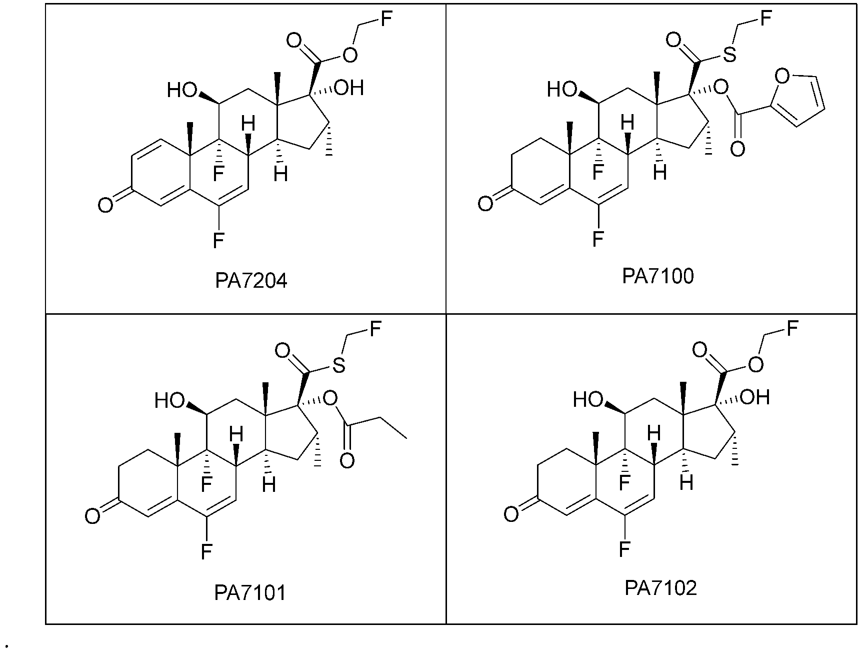

In a second aspect of the present invention, a pharmaceutical composition is provided, the pharmaceutical composition comprising: (i) the compound according to the first aspect of the present invention or a pharmaceutically acceptable salt thereof; and (ii) a pharmaceutically acceptable carrier or adjuvant.

In a third aspect of the present invention, a method for modulating the activity of a glucocorticoid receptor in a biological sample is provided, comprising the following step: contacting the glucocorticoid receptor in a biological product with the compound according to the first aspect of the present invention or a pharmaceutically acceptable salt thereof, or the pharmaceutical composition according to the second aspect of the present invention.

In a fourth aspect of the present invention, use of the compound according to the first aspect of the present invention or a pharmaceutically acceptable salt thereof, or the pharmaceutical composition according to the second aspect of the present invention in the manufacture of a medicament for alleviating or treating an inflammatory disease is provided;
preferably, the disease is selected from the group consisting of rhinitis, asthma, chronic obstructive pulmonary disease, dermatitis, arthritis, lupus, Crohn's disease, inflammatory bowel disease, celiac disease, glomerulonephritis, acne vulgaris, leukemia, and pancreatic cancer.

In a fifth aspect of the present invention, a method for treating an inflammatory disease in a patient or reducing the severity thereof, comprising the following step: administering to the patient a safe and effective amount of the compound according to the first aspect of the present invention or the pharmaceutical composition according to the second aspect of the present invention.

In another preferred embodiment, the disease is rhinitis, asthma, chronic obstructive pulmonary disease, dermatitis or arthritis.

It should be understood that within the scope of the present invention, the above technical features of the present invention and the technical features specifically described below (e.g., in the examples) can be combined with each other to form new or preferred technical solutions. Due to space limitations, they are not exhaustively listed here.

### BRIEF DESCRIPTION OF DRAWINGS

FIG. 1 shows the evaluation of the binding activity of the compound of the present application to the glucocorticoid receptor.
FIG. 2 shows the total cell count in BALF of asthma model animals after administration of the compound of the present application.
FIG. 3 shows the eosinophil count in BALF of asthma model animals after administration of the compound of the present application.
FIG. 4 shows the macrophage count in BALF of asthma model animals after administration of the compound of the present application.
FIG. 5 shows the neutrophil count in BALF of asthma model animals after administration of the compound of the present application.
FIG. 6 shows the lymphocyte count in BALF of asthma model animals after administration of the compound of the present application.

### DETAILED DESCRIPTION

Glucocorticoids have been used for nearly 60 years, and they remain the first choice for the treatment of many inflammatory and autoimmune diseases. However, long-term use of glucocorticoids can cause many adverse reactions. Therefore, the evolution of glucocorticoid drugs is driven by the need to reduce unwanted side effects while maintaining beneficial anti-inflammatory effects. Potency is a very important aspect of this evolution, because many adverse side effects are associated with high doses. Administration of high potency glucocorticoids can minimize side effects, and high potency glucocorticoids can achieve the same therapeutic effect at lower doses. This need has driven the evolution of glucocorticoids from low potency to high potency. Therefore, developing high potency glucocorticoids to minimize side effects is a key focus of attention.

After long-term and in-depth research and extensive screening, the inventors have for the first time developed a high potency glucocorticoid compound, which can achieve the same therapeutic effect at a lower dose, thereby minimizing side effects.

### Terms

For the purposes of the present invention, chemical elements are identified according to the Periodic Table of the Elements, CAS version, Handbook of Chemistry and Physics, 75th edition. In addition, the general principles of organic chemistry are described in "Organic Chemistry", Thomas Sorrell, University Science Books, Sausalito: 1999, and "March's Advanced Organic Chemistry", 5th edition, eds. Smith, M.B. and March, J., John Wiley & Sons, New York: 2001, the entire contents of which are hereby incorporated by reference.

For the purposes of the present invention, the carbon numbering of the compound of Formula I follows the accepted convention for the steroid structure. Accordingly, the compound of Formula I is numbered as follows:

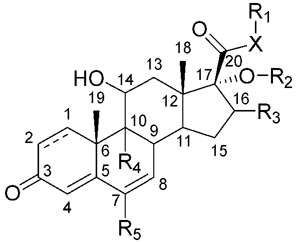

As described herein, the compound of the present invention may optionally be substituted with one or more substituents, such as those generally described above, or as exemplified by particular classes, subclasses, and species of the present invention.

As used herein, the term "hydroxyl" refers to the -OH moiety.

As used herein, the term "aliphatic" includes the terms alkyl, alkenyl, and alkynyl, each of which is optionally substituted as described below.

As used herein, "alkyl" refers to a saturated aliphatic hydrocarbon group containing 1-12 (e.g., 1-8, 1-6, or 1-4) carbon atoms. An alkyl group may be straight-chain or branched. Examples of alkyl groups include, but are not limited to, methyl, ethyl, propyl, isopropyl, butyl, isobutyl, sec-butyl, tert-butyl, n-pentyl, n-heptyl, or 2-ethylhexyl. An alkyl group may be optionally substituted with one or more substituents, such as halogen, phosphono group, cycloaliphatic group [e.g., cycloalkyl or cycloalkenyl], heterocycloaliphatic group [e.g., heterocycloalkyl or heterocycloalkenyl], aryl, heteroaryl, alkoxy, aroyl, heteroaroyl, acyl group [e.g., (aliphatic)carbonyl, (cycloaliphatic)carbonyl, or (heterocycloaliphatic)carbonyl], nitro, cyano, amido group [e.g., (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, heteroaralkylcarbonylamino, alkylaminocarbonyl, cycloalkylaminocarbonyl, heterocycloalkylaminocarbonyl, arylaminocarbonyl, or heteroarylaminocarbonyl], amino [e.g., aliphatic amino, cycloaliphatic amino, or heterocycloaliphatic amino], sulfonyl [e.g., aliphatic-SO₂-], sulfinyl, sulfanyl, sulfoxy, urea, thiourea, sulfamoyl, sulfonamide, oxo, carboxyl, carbamoyl, cycloaliphatic oxy, heterocycloaliphatic oxy, aryloxy, heteroaryloxy, aralkyloxy, heteroarylalkoxy, alkoxycarbonyl, alkylcarbonyloxy, or hydroxyl. Without limitation, some examples of substituted alkyl include carboxyalkyl (such as HOOC-alkyl, alkoxycarbonylalkyl, and alkylcarbonyloxyalkyl), cyanoalkyl, hydroxyalkyl, alkoxyalkyl, acylalkyl, aralkyl, (alkoxyaryl)alkyl, (sulfonylamino)alkyl (such as (alkyl-SO₂-amino)alkyl), aminoalkyl, amidoalkyl, (cycloaliphatic)alkyl, or haloalkyl.

As used herein, "alkenyl" refers to an aliphatic carbon group containing 2-8 (e.g., 2-8, 2-6, or 2-4) carbon atoms and at least one double bond. Like an alkyl group, an alkenyl group may be straight-chain or branched. Examples of alkenyl groups include, but are not limited to, allyl, 1- or 2-isopropenyl, 2-butenyl, and 2-hexenyl. An alkenyl group may be optionally substituted with one or more substituents, such as halogen, phosphono group, cycloaliphatic group [e.g., cycloalkyl or cycloalkenyl], heterocycloaliphatic group [e.g., heterocycloalkyl or heterocycloalkenyl], aryl, heteroaryl, alkoxy, aroyl, heteroaroyl, acyl group [e.g., (aliphatic)carbonyl, (cycloaliphatic)carbonyl, or (heterocycloaliphatic)carbonyl], nitro, cyano, amido group [e.g., (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, heteroaralkylcarbonylamino, alkylaminocarbonyl, cycloalkylaminocarbonyl, heterocycloalkylaminocarbonyl, arylaminocarbonyl, or heteroarylaminocarbonyl], amino [e.g., aliphatic amino, cycloaliphatic amino, heterocycloaliphatic amino, or aliphatic sulfonylamino], sulfonyl [e.g., alkyl-SO₂-, cycloaliphatic-SO₂-, or aryl-SO₂-], sulfinyl, sulfanyl, sulfoxy, urea, thiourea, sulfamoyl, sulfonamide, oxo, carboxyl, carbamoyl, cycloaliphatic oxy, heterocycloaliphatic oxy, aryloxy, heteroaryloxy, aralkyloxy, heteroarylalkoxy, alkoxycarbonyl, alkylcarbonyloxy, or hydroxyl. Without limitation, some examples of substituted alkenyl include cyanoalkenyl, alkoxyalkenyl, acylalkenyl, hydroxyalkenyl, aralkenyl, (alkoxyaryl)alkenyl, (sulfonylamino)alkenyl (such as (alkyl-SO₂-amino)alkenyl), aminoalkenyl, amidoalkenyl, (cycloaliphatic)alkenyl, or haloalkenyl.

As used herein, "alkynyl" refers to an aliphatic carbon group containing 2-8 (e.g., 2-12, 2-6, or 2-4) carbon atoms and having at least one triple bond. An alkynyl group may be straight-chain or branched. Examples of alkynyl groups include, but are not limited to, propargyl and butynyl. An alkynyl group may be optionally substituted with one or more substituents, such as aroyl, heteroaroyl, alkoxy, cycloalkyloxy, heterocycloalkyloxy, aryloxy, heteroaryloxy, aralkyloxy, nitro, carboxyl, cyano, halogen, hydroxyl, sulfo group, mercapto, sulfanyl [e.g., aliphatic sulfanyl or cycloaliphatic sulfanyl], sulfinyl [e.g., aliphatic sulfinyl or cycloaliphatic sulfinyl], sulfonyl [e.g., aliphatic-SO₂-, aliphatic amino-SO₂-, or cycloaliphatic-SO₂-], amido group [e.g., aminocarbonyl, alkylaminocarbonyl, alkylcarbonylamino, cycloalkylaminocarbonyl, heterocycloalkylaminocarbonyl, cycloalkylcarbonylamino, arylaminocarbonyl, arylcarbonylamino, aralkylcarbonylamino, (heterocycloalkyl)carbonylamino, (cycloalkylalkyl)carbonylamino, heteroaralkylcarbonylamino, heteroarylcarbonylamino, or heteroarylaminocarbonyl], urea, thiourea, sulfamoyl, sulfonamide, alkoxycarbonyl, alkylcarbonyloxy, cycloaliphatic group, heterocycloaliphatic group, aryl, heteroaryl, acyl group [e.g., (cycloaliphatic)carbonyl or (heterocycloaliphatic)carbonyl], amino [e.g., aliphatic amino], sulfoxy, oxo, carboxyl, carbamoyl, (cycloaliphatic)oxy, (heterocycloaliphatic)oxy, or (heteroaryl)alkoxy.

As used herein, "amido" includes "aminocarbonyl" and "carbonylamino". When used alone or in combination with another group, these terms refer to an amido group, e.g., -N(R^{x})-C(O)R^{y} or -C(O)-N(R^{x})₂ when used at a terminus, and -C(O)-N(R^{x})- or -N(R^{x})-C(O)- when used internally, wherein R^{x} and R^{y} may be hydrogen, aliphatic group, cycloaliphatic group, aryl, araliphatic group, heterocycloaliphatic group, heteroaryl, or heteroaraliphatic group. Examples of amido groups include alkylamido (such as alkylcarbonylamino or alkylaminocarbonyl), (heterocycloaliphatic)amido, (heteroaralkyl)amido, (heteroaryl)amido, (heterocycloalkyl)alkylamido, arylamido, aralkylamido, (cycloalkyl)alkylamido, or cycloalkylamido.

As used herein, "amino" refers to -NR^{x}R^{y}, wherein each of R^{x} and R^{y} is independently hydrogen, aliphatic group, cycloaliphatic group, (cycloaliphatic)aliphatic group, aryl, araliphatic group, heterocycloaliphatic group, (heterocycloaliphatic)aliphatic group, heteroaryl, carboxyl, sulfanyl, sulfinyl, sulfonyl, (aliphatic)carbonyl, (cycloaliphatic)carbonyl, ((cycloaliphatic)aliphatic)carbonyl, arylcarbonyl, (araliphatic)carbonyl, (heterocycloaliphatic)carbonyl, ((heterocycloaliphatic)aliphatic)carbonyl, (heteroaryl)carbonyl, or (heteroaraliphatic)carbonyl, each of which is defined herein and optionally substituted. Examples of amino groups include alkylamino, dialkylamino, or arylamino. When the term "amino" is not a terminal group (e.g., alkylcarbonylamino), it is represented by -NR^{x}-, wherein R^{x} has the same meaning as defined above.

As used herein, "aryl", used alone or as part of a larger moiety such as in "aralkyl", "aralkoxy", or "aryloxyalkyl", refers to monocyclic (e.g., phenyl) ring systems; bicyclic (e.g., indenyl, naphthyl, tetrahydronaphthyl, tetrahydroindenyl) ring systems; and tricyclic (e.g., fluorenyl, tetrahydrofluorenyl, or tetrahydroanthryl, anthryl) ring systems, wherein the monocyclic ring system is aromatic, or at least one ring in the bicyclic or tricyclic ring system is aromatic. Bicyclic and tricyclic groups include benzo-fused 2-3 membered carbocyclic rings. For example, benzo-fused groups include phenyl fused to two or more C₄₋₈ carbocyclic moieties. Aryl is optionally substituted with one or more substituents, including aliphatic group [e.g., alkyl, alkenyl, or alkynyl]; cycloaliphatic; (cycloaliphatic)aliphatic; heterocycloaliphatic; (heterocycloaliphatic)aliphatic; aryl; heteroaryl; alkoxy; (cycloaliphatic)oxy; (heterocycloaliphatic)oxy; aryloxy; heteroaryloxy; (araliphatic)oxy; (heteroaraliphatic)oxy; aroyl; heteroaroyl; amino; oxo (on a non-aromatic carbocyclic ring of a benzo-fused bicyclic or tricyclic aryl); nitro; carboxyl; amido group; acyl group [e.g., (aliphatic)carbonyl; (cycloaliphatic)carbonyl; ((cycloaliphatic)aliphatic)carbonyl; (araliphatic)carbonyl; (heterocycloaliphatic)carbonyl; ((heterocycloaliphatic)aliphatic)carbonyl; or (heteroaraliphatic)carbonyl]; sulfonyl [e.g., aliphatic-SO₂- or amino-SO₂-]; sulfinyl [e.g., aliphatic-S(O)- or cycloaliphatic-S(O)-]; sulfanyl [e.g., aliphatic-S-]; cyano; halogen; hydroxyl; mercapto; sulfoxy; urea, thiourea; sulfamoyl; sulfonamide; or carbamoyl. Alternatively, aryl may be unsubstituted.

Non-limiting examples of substituted aryl include haloaryl [e.g., mono-, di- (such as p-, m-dihaloaryl), and (trihalo)aryl]; (carboxy)aryl [e.g., (alkoxycarbonyl)aryl, ((aralkyl)carbonyloxy)aryl, and (alkoxycarbonyl)aryl]; (amido)aryl [e.g., (aminocarbonyl)aryl, (((alkylamino)alkyl)aminocarbonyl)aryl, (alkylcarbonyl)aminoaryl, (arylaminocarbonyl)aryl, and (((heteroaryl)amino)carbonyl)aryl]; aminoaryl [e.g., ((alkylsulfonyl)amino)aryl or ((dialkyl)amino)aryl]; (cyanoalkyl)aryl; (alkoxy)aryl; (sulfamoyl)aryl [e.g., (aminosulfonyl)aryl]; (alkylsulfonyl)aryl; (cyano)aryl; (hydroxyalkyl)aryl; ((alkoxy)alkyl)aryl; (hydroxy)aryl; ((carboxy)alkyl)aryl; (((dialkyl)amino)alkyl)aryl; (nitroalkyl)aryl; (((alkylsulfonyl)amino)alkyl)aryl; ((heterocycloaliphatic)carbonyl)aryl; ((alkylsulfonyl)alkyl)aryl; (cyanoalkyl)aryl; (hydroxyalkyl)aryl; (alkylcarbonyl)aryl; alkylaryl; (trihaloalkyl)aryl; p-amino-m-alkoxycarbonylaryl; p-amino-m-cyanoaryl; p-halo-m-aminoaryl; or (m-(heterocycloaliphatic)-o-(alkyl))aryl.

As used herein, "araliphatic", such as "aralkyl", refers to an aliphatic group (e.g., C₁₋₄ alkyl) substituted with an aryl group. "Aliphatic", "alkyl" and "aryl" are as defined herein. An example of an araliphatic group such as aralkyl is benzyl.

As used herein, "aralkyl" refers to an alkyl group (e.g., C₁₋₄ alkyl) substituted with an aryl group. Both "alkyl" and "aryl" are defined above. An example of aralkyl is benzyl. Aralkyl is optionally substituted with one or more substituents, such as aliphatic group [e.g., alkyl, alkenyl, or alkynyl, including carboxyalkyl, hydroxyalkyl, or haloalkyl such as trifluoromethyl], cycloaliphatic group [e.g., cycloalkyl or cycloalkenyl], (cycloalkyl)alkyl, heterocycloalkyl, (heterocycloalkyl)alkyl, aryl, heteroaryl, alkoxy, cycloalkyloxy, heterocycloalkyloxy, aryloxy, heteroaryloxy, aralkyloxy, heteroaralkyloxy, aroyl, heteroaroyl, nitro, carboxyl, alkoxycarbonyl, alkylcarbonyloxy, amido group [e.g., aminocarbonyl, alkylcarbonylamino, cycloalkylcarbonylamino, (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, or heteroaralkylcarbonylamino], cyano, halogen, hydroxyl, acyl group, mercapto, alkylsulfanyl, sulfoxy, urea, thiourea, sulfamoyl, sulfonamide, oxo, or carbamoyl.

As used herein, "bicyclic ring system" includes 6-12 (e.g., 8-12, or 9, 10 or 11) membered structures forming two rings, wherein the two rings share at least one common atom (e.g., 2 common atoms). Bicyclic ring systems include bicyclic aliphatic groups (e.g., bicycloalkyl or bicycloalkenyl), bicyclic heteroaliphatic groups, bicyclic aryl groups, and bicyclic heteroaryl groups.

As used herein, "cycloaliphatic" groups include "cycloalkyl" and "cycloalkenyl", each of which is optionally substituted as described below.

As used herein, "cycloalkyl" refers to a saturated carbocyclic mono- or bi- (fused or bridged) ring of 3-10 (e.g., 5-10) carbon atoms. Examples of cycloalkyl groups include cyclopropyl, cyclobutyl, cyclopentyl, cyclohexyl, cycloheptyl, adamantyl, norbornyl, cycloheptyl, octahydro-indenyl, decahydro-naphthyl, bicyclo[3.2.1]octyl, bicyclo[2.2.2]octyl, bicyclo[3.3.1]nonyl, bicyclo[3.3.2]decyl, bicyclo[2.2.2]octyl, adamantyl, or ((aminocarbonyl)cycloalkyl)cycloalkyl.

As used herein, "cycloalkenyl" refers to a non-aromatic carbocyclic ring of 3-10 (e.g., 4-8) carbon atoms having one or more double bonds. Examples of cycloalkenyl groups include cyclopentenyl, 1,4-cyclohexadienyl, cycloheptenyl, cyclooctenyl, hexahydro-indenyl, octahydro-naphthyl, cyclohexenyl, bicyclo[2.2.2]octenyl, or bicyclo[3.3.1]nonenyl.

Cycloalkyl or cycloalkenyl may be optionally substituted with one or more substituents, such as phosphono group, aliphatic group [e.g., alkyl, alkenyl, or alkynyl], cycloaliphatic group, (cycloaliphatic)aliphatic group, heterocycloaliphatic group, (heterocycloaliphatic)aliphatic group, aryl, heteroaryl, alkoxy, (cycloaliphatic)oxy, (heterocycloaliphatic)oxy, aryloxy, heteroaryloxy, (araliphatic)oxy, (heteroaraliphatic)oxy, aroyl, heteroaroyl, amino, amido group [e.g., (aliphatic)carbonylamino, (cycloaliphatic)carbonylamino, ((cycloaliphatic)aliphatic)carbonylamino, (aryl)carbonylamino, (araliphatic)carbonylamino, (heterocycloaliphatic)carbonylamino, ((heterocycloaliphatic)aliphatic)carbonylamino, (heteroaryl)carbonylamino, or (heteroaraliphatic)carbonylamino], nitro, carboxyl [e.g., HOOC-, alkoxycarbonyl, or alkylcarbonyloxy], acyl group [e.g., (cycloaliphatic)carbonyl, ((cycloaliphatic)aliphatic)carbonyl, (araliphatic)carbonyl, (heterocycloaliphatic)carbonyl, ((heterocycloaliphatic)aliphatic)carbonyl, or (heteroaraliphatic)carbonyl], cyano, halogen, hydroxyl, mercapto, sulfonyl [e.g., alkyl-SO₂- and aryl-SO₂-], sulfinyl [e.g., alkyl-S(O)-], sulfanyl [e.g., alkyl-S-], sulfoxy, urea, thiourea, sulfamoyl, sulfonamide, oxo, or carbamoyl.

As used herein, the term "heterocycloaliphatic" includes heterocycloalkyl and heterocycloalkenyl, each of which is optionally substituted as described below.

As used herein, "heterocycloalkyl" refers to a mono- or bicyclic (fused or bridged) saturated ring structure of 3-10 members (e.g., 5- to 10-membered mono- or bicyclic), wherein one or more ring atoms are heteroatoms (e.g., N, O, S, or a combination thereof). Examples of heterocycloalkyl groups include piperidinyl, piperazinyl, tetrahydropyranyl, tetrahydrofuranyl, 1,4-dioxolanyl, 1,4-dithianyl, 1,3-dioxolanyl, oxazolidinyl, isoxazolidinyl, morpholinyl, thiomorpholinyl, octahydrobenzofuranyl, octahydrochromenyl, octahydrothiochromenyl, octahydroindolyl, octahydropyridinyl, decahydroquinolinyl, octahydrobenzo[b]thiophenyl, 2-oxa-bicyclo[2.2.2]octyl, 1-aza-bicyclo[2.2.2]octyl, 3-aza-bicyclo[3.2.1]octyl, and 2,6-dioxa-tricyclo[3.3.1.03,7]nonyl. A monocyclic heterocycloalkyl may be fused with a phenyl moiety to form a structure such as tetrahydroisoquinoline, which would be classified as heteroaryl.

As used herein, "heterocycloalkenyl" refers to a mono- or bicyclic (e.g., 5- to 10-membered mono- or bicyclic) non-aromatic ring structure having one or more double bonds, and wherein one or more ring atoms are heteroatoms (e.g., N, O or S). Monocyclic and bicyclic heterocycloaliphatic groups are numbered according to standard chemical nomenclature.

Heterocycloalkyl or heterocycloalkenyl may be optionally substituted with one or more substituents, such as phosphono group, aliphatic group [e.g., alkyl, alkenyl, or alkynyl], cycloaliphatic group, (cycloaliphatic)aliphatic group, heterocycloaliphatic group, (heterocycloaliphatic)aliphatic group, aryl, heteroaryl, alkoxy, (cycloaliphatic)oxy, (heterocycloaliphatic)oxy, aryloxy, heteroaryloxy, (araliphatic)oxy, (heteroaraliphatic)oxy, aroyl, heteroaroyl, amino, amido group [e.g., (aliphatic)carbonylamino, (cycloaliphatic)carbonylamino, ((cycloaliphatic)aliphatic)carbonylamino, (aryl)carbonylamino, (araliphatic)carbonylamino, (heterocycloaliphatic)carbonylamino, ((heterocycloaliphatic)aliphatic)carbonylamino, (heteroaryl)carbonylamino, or (heteroaraliphatic)carbonylamino], nitro, carboxyl [e.g., HOOC-, alkoxycarbonyl, or alkylcarbonyloxy], acyl group [e.g., (cycloaliphatic)carbonyl, ((cycloaliphatic)aliphatic)carbonyl, (araliphatic)carbonyl, (heterocycloaliphatic)carbonyl, ((heterocycloaliphatic)aliphatic)carbonyl, or (heteroaraliphatic)carbonyl], nitro, cyano, halogen, hydroxyl, mercapto, sulfonyl [e.g., alkylsulfonyl or arylsulfonyl], sulfinyl [e.g., alkylsulfinyl], sulfanyl [e.g., alkylsulfanyl], sulfoxy, urea, thiourea, sulfamoyl, sulfonamide, oxo, or carbamoyl.

As used herein, "heteroaryl" refers to a monocyclic, bicyclic, or tricyclic ring system having 4 to 15 ring atoms, wherein one or more ring atoms are heteroatoms (e.g., N, O, S, or a combination thereof), and wherein the monocyclic ring system is aromatic, or at least one ring in the bicyclic or tricyclic ring system is aromatic. Heteroaryl includes benzo-fused ring systems having 2 to 3 rings. For example, benzo-fused groups include benzo groups fused with one or two 4- to 8-membered heterocycloaliphatic moieties (e.g., indolizinyl, indolyl, isoindolyl, 3H-indolyl, indolinyl, benzo[b]furanyl, benzo[b]thiophenyl, quinolinyl, or isoquinolinyl). Some examples of heteroaryl are azetidinyl, pyridinyl, 1H-indolyl, furanyl, pyrrolyl, thiophenyl, thiazolyl, oxazolyl, imidazolyl, tetrazolyl, benzofuranyl, isoquinolinyl, benzothiazolyl, xanthene, thioxanthene, phenothiazine, indoline, benzo[1,3]dioxole, benzo[b]furanyl, benzo[b]thiophenyl, indolyl, benzimidazolyl, benzothiazolyl, purinyl, cinnolinyl, quinolinyl, quinazolinyl, cinnolinyl, phthalazinyl, quinazolinyl, quinoxalinyl, isoquinolinyl, 4H-quinolizinyl, benzo-1,2,5-thiadiazolyl, or 1,8-naphthyridinyl.

Without limitation, monocyclic heteroaryl includes furanyl, thiophenyl, 2H-pyrrolyl, pyrrolyl, oxazolyl, thiazolyl, imidazolyl, pyrazolyl, isoxazolyl, isothiazolyl, 1,3,4-thiadiazolyl, 2H-pyranyl, 4H-pyranyl, pyridinyl, pyridazinyl, pyrimidinyl, pyrazolyl, pyrazinyl, or 1,3,5-triazinyl. Monocyclic heteroaryl is numbered according to standard chemical nomenclature.

Without limitation, bicyclic heteroaryl includes indolizinyl, indolyl, isoindolyl, 3H-indolyl, indolinyl, benzo[b]furanyl, benzo[b]thiophenyl, quinolinyl, isoquinolinyl, indolizinyl, isoindolyl, indolyl, benzo[b]furanyl, benzo[b]thiophenyl, indazolyl, benzimidazolyl, benzothiazolyl, purinyl, 4H-quinolizinyl, quinolinyl, isoquinolinyl, cinnolinyl, phthalazinyl, quinazoline, quinoxalinyl, 1,8-naphthyridinyl, or pteridinyl. Bicyclic heteroaryl is numbered according to standard chemical nomenclature.

Heteroaryl is optionally substituted with one or more substituents, such as aliphatic group [e.g., alkyl, alkenyl, or alkynyl]; cycloaliphatic; (cycloaliphatic)aliphatic; heterocycloaliphatic; (heterocycloaliphatic)aliphatic; aryl; heteroaryl; alkoxy; (cycloaliphatic)oxy; (heterocycloaliphatic)oxy; aryloxy; heteroaryloxy; (araliphatic)oxy; (heteroaraliphatic)oxy; aroyl; heteroaroyl; amino; oxo (on a non-aromatic carbocyclic or heterocyclic ring of a bicyclic or tricyclic heteroaryl); carboxyl; amido group; acyl group [e.g., aliphatic carbonyl; (cycloaliphatic)carbonyl; ((cycloaliphatic)aliphatic)carbonyl; (araliphatic)carbonyl; (heterocycloaliphatic)carbonyl; ((heterocycloaliphatic)aliphatic)carbonyl; or (heteroaraliphatic)carbonyl]; sulfonyl [e.g., aliphatic sulfonyl or aminosulfonyl]; sulfinyl [e.g., aliphatic sulfinyl]; sulfanyl [e.g., aliphatic sulfanyl]; nitro; cyano; halogen; hydroxyl; mercapto; sulfoxy; urea; thiourea; sulfamoyl; sulfonamide; or carbamoyl. Alternatively, heteroaryl may be unsubstituted.

Non-limiting examples of substituted heteroaryl include (halo)heteroaryl [e.g., mono- and di-(halo)heteroaryl]; (carboxy)heteroaryl [e.g., (alkoxycarbonyl)heteroaryl]; cyanoheteroaryl; aminoheteroaryl [e.g., ((alkylsulfonyl)amino)heteroaryl and ((dialkyl)amino)heteroaryl]; (amido)heteroaryl [e.g., aminocarbonylheteroaryl, ((alkylcarbonyl)amino)heteroaryl, (((alkylamino)alkyl)aminocarbonyl)heteroaryl, (((heteroaryl)amino)carbonyl)heteroaryl, ((heterocycloaliphatic)carbonyl)heteroaryl, and ((alkylcarbonyl)amino)heteroaryl]; (cyanoalkyl)heteroaryl; (alkoxy)heteroaryl; (sulfamoyl)heteroaryl [e.g., (aminosulfonyl)heteroaryl]; (sulfonyl)heteroaryl [e.g., (alkylsulfonyl)heteroaryl]; (hydroxyalkyl)heteroaryl; (alkoxyalkyl)heteroaryl; (hydroxy)heteroaryl; ((carboxy)alkyl)heteroaryl; (((dialkyl)amino)alkyl)heteroaryl; (heterocycloaliphatic)heteroaryl; (cycloaliphatic)heteroaryl; (nitroalkyl)heteroaryl; (((alkylsulfonyl)amino)alkyl)heteroaryl; ((alkylsulfonyl)alkyl)heteroaryl; (cyanoalkyl)heteroaryl; (acyl)heteroaryl [e.g., (alkylcarbonyl)heteroaryl]; (alkyl)heteroaryl; or (haloalkyl)heteroaryl [e.g., trihaloalkylheteroaryl].

As used herein, "heteroaraliphatic" (such as heteroaralkyl) refers to an aliphatic group (e.g., C₁₋₄ alkyl) substituted with a heteroaryl group. "Aliphatic", "alkyl" and "heteroaryl" are defined above.

As used herein, "heteroaralkyl" refers to an alkyl group (e.g., C₁₋₄ alkyl) substituted with a heteroaryl group. Both "alkyl" and "heteroaryl" are defined above. Heteroaralkyl is optionally substituted with one or more substituents, such as alkyl (including carboxyalkyl, hydroxyalkyl, and haloalkyl such as trifluoromethyl), alkenyl, alkynyl, cycloalkyl, (cycloalkyl)alkyl, heterocycloalkyl, (heterocycloalkyl)alkyl, aryl, heteroaryl, alkoxy, cycloalkyloxy, heterocycloalkyloxy, aryloxy, heteroaryloxy, aralkyloxy, heteroaralkyloxy, aroyl, heteroaroyl, nitro, carboxyl, alkoxycarbonyl, alkylcarbonyloxy, aminocarbonyl, alkylcarbonylamino, cycloalkylcarbonylamino, (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, heteroaralkylcarbonylamino, cyano, halogen, hydroxyl, acyl group, mercapto, alkylsulfanyl, sulfoxy, urea, thiourea, sulfamoyl, sulfonamide, oxo, or carbamoyl.

As used herein, "cyclic moiety" and "cyclic group" refer to mono-, bi- and tricyclic ring systems, including cycloaliphatic, heterocycloaliphatic, aryl or heteroaryl, each of which is defined above.

As used herein, "bridged bicyclic ring system" refers to a bicyclic heterocycloaliphatic ring system or a bicyclic cycloaliphatic ring system wherein the rings are bridged. Examples of bridged bicyclic ring systems include, but are not limited to, adamantyl, norbornyl, bicyclo[3.2.1]octyl, bicyclo[2.2.2]octyl, bicyclo[3.3.1]nonyl, bicyclo[3.3.2]decyl, 2-oxabicyclo[2.2.2]octyl, 1-azabicyclo[2.2.2]octyl, 3-azabicyclo[3.2.1]octyl, and 2,6-dioxa-tricyclo[3.3.1.03,7]nonyl. A bridged bicyclic ring system may be optionally substituted with one or more substituents, such as alkyl (including carbonylalkyl, hydroxyalkyl, and haloalkyl such as trifluoromethyl), alkenyl, alkynyl, cycloalkyl, (cycloalkyl)alkyl, heterocycloalkyl, (heterocycloalkyl)alkyl, aryl, heteroaryl, alkoxy, cycloalkyloxy, heterocycloalkyloxy, aralkyloxy, heteroaryloxy, aralkyloxy, heteroaralkyloxy, aroyl, heteroaroyl, nitro, carboxyl, alkoxycarbonyl, alkylcarbonyloxy, aminocarbonyl, alkylcarbonylamino, cycloalkylcarbonylamino, (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, heteroaralkylcarbonylamino, cyano, halogen, hydroxyl, acyl group, mercapto, alkylsulfanyl, sulfoxy, urea, thiourea, sulfamoyl, sulfonamide, oxo, or carbamoyl.

As used herein, acyl refers to formyl or R^{x}-C(O)- (such as alkyl-C(O)-, also referred to as alkylcarbonyl), wherein R^{x} and alkyl are defined above. Acetyl and pivaloyl are examples of acyl groups. Acetyl and pivaloyl are examples of acyl groups.

As used herein, "aroyl" or "heteroaroyl" refers to aryl-C(O)- or heteroaryl-C(O)-. The aryl and heteroaryl moieties of aroyl or heteroaroyl are optionally substituted as defined above.

As used herein, an "alkoxy" group refers to an alkyl-O- group, wherein "alkyl" is defined above.

As used herein, "carbamoyl" refers to a group having the structure -O-CO-NR^{x}R^{y} or -NR^{x}-CO-O-R^{z}, wherein R^{x} and R^{y} are defined above, and R^{z} may be aliphatic group, aryl, araliphatic group, heterocycloaliphatic group, heteroaryl, or heteroaraliphatic group.

As used herein, "carboxyl" when used as a terminal group refers to -COOH, -COOR^{x}, -OC(O)H, -OC(O)R^{x}; or when used as an internal group refers to -OC(O)- or -C(O)O-.

As used herein, a "haloaliphatic" group refers to an aliphatic group substituted with 1-3 halogen atoms. For example, the term haloalkyl includes the groups -CF₃, -CHF₂ and -CH₂F.

As used herein, "mercapto" refers to -SH.

As used herein, "sulfo group" when used at a terminus refers to -SO₃H or -SO₃R^{x}, or when used internally refers to -S(O)₃-O-.

As used herein, a "sulfonamide" group when used at a terminus refers to -S(O)₂-NR^{x}R^{y}, and when used internally refers to -NR^{x}-S(O)₂-NR^{y}-, wherein R^{x}R^{y} and R^{z} are defined above.

As used herein, "sulfamoyl" refers to the structure -O-S(O)₂-NR^{x}R^{y}, wherein R^{x} and R^{y} are defined above.

As used herein, a "sulfonamide" group when used at a terminus refers to -S(O)₂-NR^{y}R^{z} or -NR^{x}-S(O)₂-R^{z}; or when used internally refers to -S(O)₂-NR^{x}- or -NR^{x}-S(O)₂-, wherein R^{x}R^{y} and R^{z} are as defined above.

As used herein, "sulfanyl" when used at a terminus refers to -S-R^{x}, and when used internally refers to -S-, wherein R^{x} is defined above. Examples of sulfanyl include aliphatic-S-, cycloaliphatic-S-, aryl-S-, and the like.

As used herein, "sulfinyl" when used at a terminus refers to -S(O)-R^{x}, and when used internally refers to -S(O)-, wherein R^{x} is defined above. Exemplary sulfinyl groups include aliphatic-S(O)-, aryl-S(O)-, (cycloaliphatic(aliphatic))-S(O)-, cycloalkyl-S(O)-, heterocycloaliphatic-S(O)-, heteroaryl-S(O)-, and the like.

As used herein, "sulfonyl" when used at a terminus refers to -S(O)₂-R^{x}, and when used internally refers to -S(O)₂-, wherein R^{x} is defined above. Exemplary sulfonyl groups include aliphatic-S(O)₂-, aryl-S(O)₂-, (cycloaliphatic(amido(aliphatic)))-S(O)₂-, cycloaliphatic-S(O)₂-, heterocycloaliphatic-S(O)₂-, heteroaryl-S(O)₂-, and the like.

As used herein, "sulfonyloxy" when used at a terminus refers to -O-S(O)-R^{x} or -S(O)-R^{x}, and when used internally refers to -O-S(O)- or -S(O)-O-, wherein R^{x} is defined above.

As used herein, "halogen" or "halo" group refers to fluorine, chlorine, bromine or iodine.

As used herein, "alkoxycarbonyl", encompassed by the term carboxyl, used alone or in combination with another group, refers to a group such as alkyl-O-C(O)-.

As used herein, "alkoxyalkyl" refers to an alkyl group, such as alkyl-O-alkyl-, wherein alkyl is defined above. As used herein, "carbonyl" refers to -C(O)-.

As used herein, "oxo" refers to =O.

As used herein, the term "phosphono group" refers to hypophosphite and phosphate esters. Examples of hypophosphite and phosphate esters include -P(O)(RP)₂, wherein RP is aliphatic group, alkoxy, aryloxy, heteroaryloxy, (cycloaliphatic)oxy, (heterocycloaliphatic)oxy, aryl, heteroaryl, cycloaliphatic, or amino.

As used herein, "aminoalkyl" refers to the structure (R^{x})2N-alkyl-.

As used herein, "cyanoalkyl" refers to the structure (NC)-alkyl-.

As used herein, "ureido" refers to the structure -NR^{x}-CO-NR^{x}R^{y}, and "thioureido" when used at a terminus refers to the structure -NR^{x}-CS-NR^{x}R^{y} and when used internally refers to -NR^{x}-CO-NR^{y}- or -NR^{x}-CS-NR^{y}-, wherein R^{x}R^{y} and R^{z} are defined above.

As used herein, "guanidino" refers to the structure -N=C(N(R^{x}R^{y}))N(R^{x}R^{y}) or -NR^{x}-C(=NR^{x})NR^{x}R^{y}, wherein R^{x} and R^{y} are defined above.

As used herein, the term "amidino" refers to the structure -C(=NR^{x})N(R^{x}R^{y}), wherein R^{x} and R^{y} are defined above.

As used herein, the term "plurality" refers to a positive integer of 2, 3, 4, 5 or greater than 5.

The terms "at a terminus" and "internally" refer to the position of a group within a substituent. A group is terminal when it is present at the terminus of a substituent without further bonding to the rest of the chemical structure. Carboxyalkyl, i.e., R^{x}O(O)C-alkyl, is an example of a carboxyl group used at a terminus. A group is internal when it is present in the middle of a substituent of a chemical structure. Alkylcarboxyl (e.g., alkyl-C(O)O- or alkyl-OC(O)-) and alkylcarboxyaryl (e.g., alkyl-C(O)O-aryl- or alkyl-O(CO)-aryl-) are examples of carboxyl groups used internally.

As used herein, "aliphatic chain" refers to a branched or straight-chain aliphatic group (e.g., alkyl, alkenyl, or alkynyl). In some embodiments, a straight-chain aliphatic chain has the structure -[CH₂]_{V}-, wherein v is 1-12. In some embodiments, a branched aliphatic chain is a straight-chain aliphatic chain substituted with one or more aliphatic groups. A branched aliphatic chain has the structure -[CQQ]_{V}-, wherein Q is independently hydrogen or an aliphatic group; however, in at least one instance, Q shall be an aliphatic group. The term aliphatic chain includes alkyl chains, alkenyl chains, and alkynyl chains, wherein alkyl, alkenyl, and alkynyl are as defined above.

The phrase "optionally substituted" is used interchangeably with the phrase "substituted or unsubstituted". As described herein, the compounds of the present invention may optionally be substituted with one or more substituents, such as those generally set forth above, or as exemplified by the particular classes, subclasses and species of the present invention. As described herein, the variables R₁, R₂, R₃, R₄, R₅ and X, and other variables contained in the formulae described herein, encompass specific groups, such as alkyl and aryl. Unless otherwise noted, each of the specific groups for these variables may optionally be substituted with one or more substituents described herein. Each substituent of a specific group is further optionally substituted with 1 to 3 halogens, cyano, oxo, alkoxy, hydroxyl, amino, nitro, aryl, cycloaliphatic group, heterocycloaliphatic group, heteroaryl, haloalkyl, and alkyl. For example, an alkyl group may be substituted with alkylsulfanyl, and the alkylsulfanyl may be optionally substituted with 1 to 3 halogens, cyano, oxo, alkoxy, hydroxyl, amino, nitro, aryl, heteroaryl, haloalkyl, and alkyl. As an additional example, the cycloalkyl moiety of (cycloalkyl)carbonylamino may be optionally substituted with 1 to 3 halogens, cyano, alkoxy, hydroxyl, nitro, haloalkyl, and alkyl. When two alkoxy groups are bonded to the same atom or adjacent atoms, the two alkoxy groups may form a ring together with the atom(s) to which they are bonded.

In general, the term "substituted", whether preceded by the term "optionally" or not, refers to the replacement of hydrogen radicals in a given structure with the given substituent. Specific substituents are described above in the definitions and in the following description of compounds and examples thereof. Unless otherwise indicated, an optionally substituted group may have a substituent at each substitutable position of the group, and when more than one position in any given structure can be substituted with more than one substituent selected from a specified group, the substituents may be the same or different at each position. A ring substituent, such as heterocycloalkyl, may be bonded to another ring (such as cycloalkyl) to form a spiro-bicyclic ring system, e.g., the two rings share one common atom. As one of ordinary skill in the art will recognize, the combinations of substituents contemplated by the present invention are those that result in the formation of stable or chemically feasible compounds. Unless otherwise stated, the structures depicted herein are also meant to include all isomeric (e.g., enantiomeric, diastereomeric, and geometric (or conformational)) forms of the structure; for example, the R and S configurations for each asymmetric center, (Z) and (E) double bond isomers, and (Z) and (E) conformational isomers. Therefore, single stereochemical isomers as well as enantiomeric, diastereomeric, and geometric (or conformational) mixtures of the compounds of the present invention are within the scope of the present invention.

Additionally, unless otherwise stated, the structures depicted herein are also meant to include compounds that differ only in the presence of one or more isotopically enriched atoms. For example, compounds having the structures of the present invention except for the replacement of hydrogen by deuterium or tritium, or the replacement of carbon by ¹³C- or ¹⁴C-enriched carbon are within the scope of this invention. Such compounds are useful, for example, as analytical tools or probes in biological assays, or as therapeutic agents.

### Active Ingredient

As used herein, "compound of the present invention" refers to a compound represented by Formula I or a pharmaceutically acceptable salt thereof. 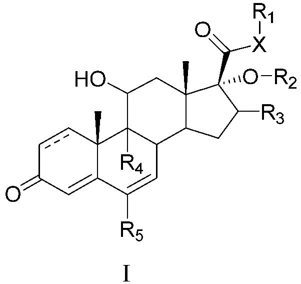 wherein:
X is -O- or -S-;
---is a bond or is absent;
R₁ is selected from hydrogen, C₁₋₆ alkyl, cycloalkyl, aryl, heteroaryl, aralkyl, heteroaralkyl or heterocycloalkyl, any of which is optionally substituted;
R₂ is -L-R'-, wherein L is a bond or -C(O)-, and R' is selected from hydrogen, C₁₋₆ alkyl, cycloalkyl, aryl, heteroaryl, aralkyl, heteroaralkyl or heterocycloalkyl, wherein R' is optionally substituted;
R₃ is hydrogen or optionally substituted C₁₋₆ alkyl;
R₄ and R₅ are each independently hydrogen, halogen, or C₁₋₄ alkyl.

In some embodiments, R₁ is hydrogen, C₁₋₆ alkyl, cycloalkyl, or aryl, wherein the alkyl, cycloalkyl or aryl is optionally substituted. For example, R₁ is alkyl, cycloalkyl or aryl, any of which is optionally substituted with halogen.

In other embodiments, R₁ is hydrogen or C₁₋₆ alkyl, wherein the C₁₋₆ alkyl is optionally substituted with 1-3 halogens.

In some embodiments, R₁ is methyl, ethyl, propyl, isopropyl, butyl, isobutyl, or tert-butyl, any of which is optionally substituted with 1-3 halogens. For example, R₁ is fluoromethyl.

In other embodiments, R₁ is unsubstituted C₁₋₆ alkyl. For example, R₁ is methyl, ethyl, propyl, isopropyl, butyl, isobutyl, or tert-butyl. In certain cases, R₁ is ethyl. In certain cases, R₁ is isopropyl. In certain cases, R₁ is tert-butyl. In certain cases, R₁ is hydrogen.

In some embodiments, R₁ is optionally substituted 3- to 7-membered cycloalkyl, phenyl, or naphthyl, any of which is optionally substituted.

In some embodiments, L is -C(O)-, and R' is hydrogen, C₁₋₆ alkyl, aralkyl, heteroaryl, or heteroaralkyl, wherein R₂ is optionally substituted with halogen, CN, carboxyl or oxo group. For example, L is -C(O)-, and R₂ is heteroaryl.

In other embodiments, R₂ is

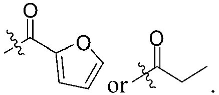

In some embodiments, R₃ is optionally substituted C₁₋₆ alkyl. For example, R₃ is methyl, ethyl, propyl, isopropyl, butyl, isobutyl, or tert-butyl, any of which is optionally substituted.

In other embodiments, R₃ is hydrogen.

In some embodiments, X is -O-.

In some embodiments, X is -S-.

In some embodiments, --- is a bond.

In some embodiments, --- is absent.

In some embodiments, R₄ and R₅ are each independently hydrogen or halogen. For example, both R₄ and R₅ are F.

In some embodiments, the compound of Formula I is selected from the compounds in Table 1 below.

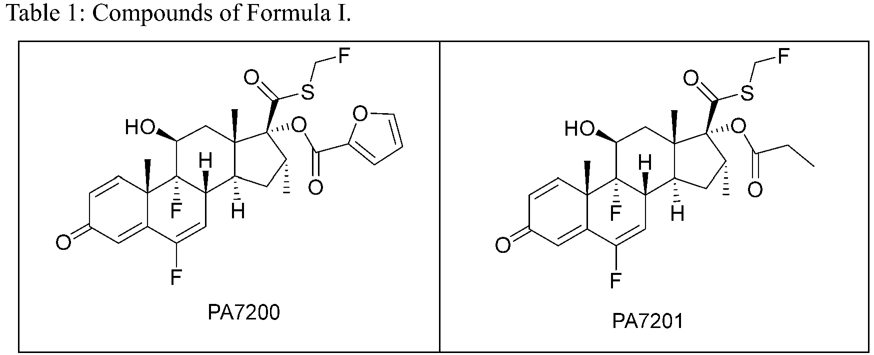

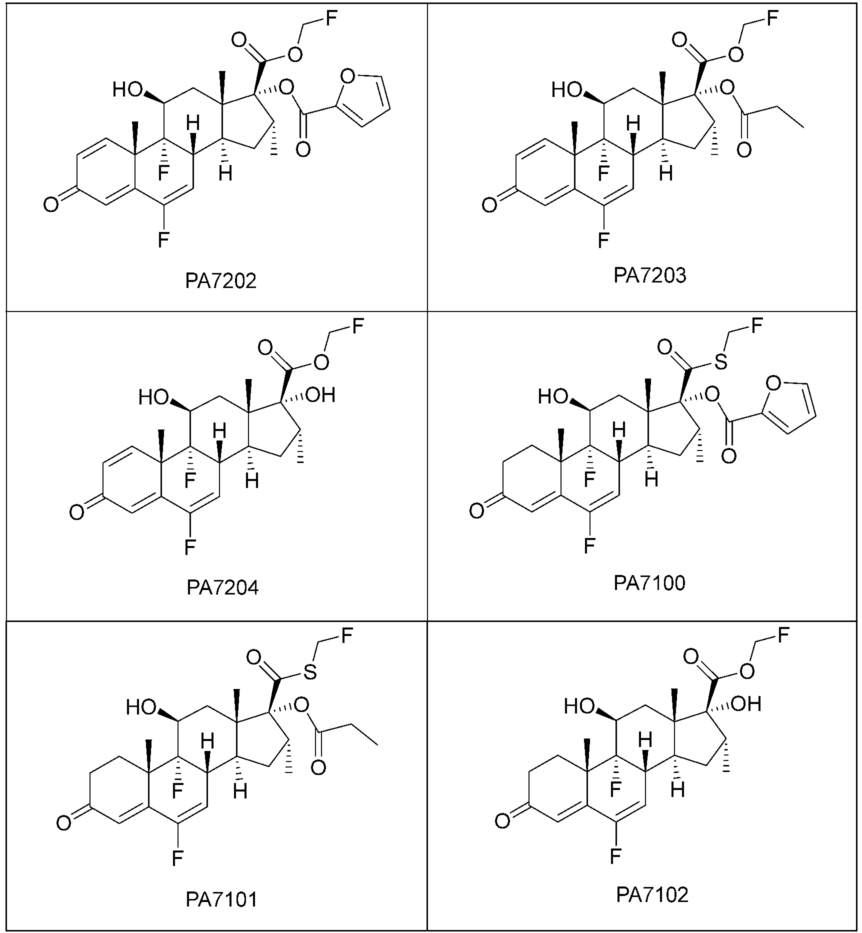

### Pharmaceutical Compositions

In one aspect of the present invention, pharmaceutically acceptable compositions, i.e., pharmaceutical compositions, are provided, wherein these compositions comprise any compound as described herein, and optionally comprise a pharmaceutically acceptable carrier, adjuvant or vehicle. In certain embodiments, these compositions optionally further comprise one or more additional therapeutic agents.

It should also be understood that certain compounds of the present invention may exist in free form for therapeutic use, or where appropriate, as a pharmaceutically acceptable derivative or prodrug thereof. According to the present invention, pharmaceutically acceptable derivatives or prodrugs include, but are not limited to, pharmaceutically acceptable salts, esters, salts of such esters, or any other adduct or derivative that, upon administration to a patient in need thereof, is capable of directly or indirectly providing a compound as otherwise described herein, or a metabolite or residue thereof.

As used herein, the term "pharmaceutically acceptable salt" refers to those salts which, within the scope of sound medical judgment, are suitable for use in contact with the tissues of humans and lower animals without undue toxicity, irritation, allergic response and the like, and are commensurate with a reasonable benefit/risk ratio. "Pharmaceutically acceptable salt" means any non-toxic salt or salt of an ester of a compound of the present invention that, upon administration to a recipient, is capable of directly or indirectly providing a compound of the present invention or an inhibitory active metabolite or residue thereof.

Pharmaceutically acceptable salts are well known in the art. For example, S. M. Berge et al. describe pharmaceutically acceptable salts in detail in J. Pharmaceutical Sciences, 1977, 66, 1-19, which is incorporated herein by reference. The pharmaceutically acceptable salts of the compounds of the present invention include those derived from suitable inorganic and organic acids and bases. Examples of pharmaceutically acceptable non-toxic acid addition salts are salts of an amino group formed with inorganic acids such as hydrochloric acid, hydrobromic acid, phosphoric acid, sulfuric acid and perchloric acid, or with organic acids such as acetic acid, oxalic acid, maleic acid, tartaric acid, citric acid, succinic acid or malonic acid, or by using other methods used in the art such as ion exchange.

Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecyl sulfate, ethanedisulfonate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate, and the like. Salts derived from appropriate bases include alkali metal, alkaline earth metal, ammonium and N⁺(C₁₋₄ alkyl)₄ salts. The present invention also contemplates quaternization of any basic nitrogen-containing groups of the compounds disclosed herein. Water- or oil-soluble or dispersible products may be obtained by such quaternization. Representative alkali or alkaline earth metal salts include sodium, lithium, potassium, calcium, magnesium, and the like. Where appropriate, additional pharmaceutically acceptable salts include non-toxic ammonium, quaternary ammonium and amine cations formed using counterions such as halide, hydroxide, carboxylate, sulfate, phosphate, nitrate, lower alkyl sulfonate and aryl sulfonate.

As stated above, the pharmaceutically acceptable compositions of the present invention additionally comprise a pharmaceutically acceptable carrier, adjuvant or vehicle, which as used herein includes any and all solvents, diluents, or other liquid vehicles, dispersion or suspension aids, surface-active agents, isotonic agents, thickening or emulsifying agents, preservatives, solid binders, lubricants and the like, as suited to the particular dosage form desired. Remington's Pharmaceutical Sciences, Sixteenth Edition, E. W. Martin (Mack Publishing Co., Easton, Pa., 1980) discloses various carriers used in formulating pharmaceutically acceptable compositions and known techniques for the preparation thereof. Except insofar as any conventional carrier medium is incompatible with the compounds of the present invention, such as by producing any undesirable biological effect or otherwise interacting in a deleterious manner with any other component(s) of the pharmaceutically acceptable composition, its use is contemplated to be within the scope of the present invention. Some examples of materials that can serve as pharmaceutically acceptable carriers include, but are not limited to, ion exchangers, alumina, aluminum stearate, lecithin, serum proteins such as human serum albumin, buffer substances such as phosphates, glycine, sorbic acid or potassium sorbate, partial glyceride mixtures of saturated vegetable fatty acids, water, salts or electrolytes such as protamine sulfate, disodium hydrogen phosphate, potassium hydrogen phosphate, sodium chloride, zinc salts, colloidal silica, magnesium trisilicate, polyvinyl pyrrolidone, polyacrylates, waxes, polyethylene-polyoxypropylene-block polymers, lanolin, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols such as propylene glycol or polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethanol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

### Use and Administration of Compounds and Pharmaceutical Compositions

In another aspect, the present invention provides a method for treating inflammation in a subject or reducing the severity thereof, the method comprising administering to a subject in need thereof, preferably a mammal, an effective amount of a composition comprising a compound of Formula I. The present invention also provides a method for treating an inflammatory disease in a subject or reducing the severity thereof, the method comprising administering to a subject in need thereof, preferably a mammal, an effective amount of a composition comprising a compound of Formula I.

In another aspect, the present invention provides a method for treating a condition, disease or disorder involving the glucocorticoid receptor or reducing the severity thereof. In certain embodiments, the present invention provides a method for treating a condition, disease or disorder involving deficient glucocorticoid receptor activity, the method comprising administering to a subject in need thereof, preferably a mammal, an effective amount of a composition comprising a compound of Formula I. In certain embodiments, the present invention provides a method for treating an inflammatory condition, disease or disorder in a subject, wherein the subject has normal glucocorticoid receptor activity, the method comprising administering to a subject in need thereof, preferably a mammal, an effective amount of a composition comprising a compound of Formula I.

In another aspect, the present invention provides a method for treating a condition, disease or disorder involving the glucocorticoid receptor or reducing the severity thereof, wherein the condition, disease or disorder is selected from rhinitis, asthma, chronic obstructive pulmonary disease, dermatitis, arthritis, lupus, Crohn's disease, inflammatory bowel disease, celiac disease, glomerulonephritis, acne vulgaris, leukemia and pancreatic cancer.

According to the present invention, an "effective amount" of the compound or pharmaceutically acceptable composition is an amount effective to treat or reduce the severity of one or more diseases, disorders or conditions as described above.

The compounds and compositions according to the methods of the present invention may be administered using any amount and any route of administration effective to treat or reduce the severity of one or more diseases, disorders or conditions as described above.

The exact amount required will vary from subject to subject, depending on the species, age and general condition of the subject, the severity of the infection, the particular agent, its mode of administration, and the like. The compounds of the present invention are preferably formulated in dosage unit form for ease of administration and uniformity of dosage. The expression "dosage unit form" as used herein refers to a physically discrete unit of pharmaceutical agent appropriate for the patient to be treated. It should be understood, however, that the total daily usage of the compounds and compositions of the present invention will be decided by the attending physician within the scope of sound medical judgment. The specific effective dose level for any particular patient or organism will depend upon a variety of factors, including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed, and like factors well known in the medical arts. As used herein, the term "patient" refers to an animal, preferably a mammal, and most preferably a human.

Depending on the severity of the infection being treated, the pharmaceutically acceptable compositions of the present invention may be administered to humans and other animals orally, rectally, parenterally, intracisternally, intravaginally, intraperitoneally, topically (as by powders, ointments, drops or patches), buccally, as an oral or nasal spray, or the like. In certain embodiments, the compounds of the present invention may be administered orally or parenterally at dosage levels of about 0.01 mg/Kg to about 0.5 mg/Kg, preferably from about 0.5 mg/Kg to 20 mg/Kg of subject body weight per day, one or more times a day, to obtain the desired therapeutic effect.

Liquid dosage forms for oral administration include, but are not limited to, pharmaceutically acceptable emulsions, microemulsions, solutions, suspensions, syrups and elixirs. In addition to the active compounds, these liquid dosage forms may contain inert diluents commonly used in the art, such as, for example, water or other solvents, solubilizing agents and emulsifiers, such as ethanol, isopropyl alcohol, ethyl carbonate, ethyl acetate, benzyl alcohol, benzyl benzoate, propylene glycol, 1,3-butylene glycol, dimethylformamide, oils (in particular, cottonseed oil, peanut oil, corn oil, germ oil, olive oil, castor oil, and sesame oil), glycerol, tetrahydrofurfuryl alcohol, polyethylene glycols, and sorbitan fatty acid esters, and mixtures thereof. Besides inert diluents, these oral compositions can also include adjuvants such as wetting agents, emulsifying and suspending agents, sweetening, flavoring, and perfuming agents.

Injectable preparations, for example, sterile injectable aqueous or oleaginous suspensions, may be formulated according to the known art using suitable dispersing or wetting agents and suspending agents. The sterile injectable preparation may also be a sterile injectable solution, suspension or emulsion in a non-toxic parenterally acceptable diluent or solvent, for example, as a solution in 1,3-butanediol. Acceptable vehicles and solvents that may be employed include water, Ringer's solution, U.S.P. and isotonic sodium chloride solution. In addition, sterile, fixed oils are conventionally employed as a solvent or suspending medium. For this purpose, any bland fixed oil may be employed, including synthetic mono- or diglycerides. In addition, fatty acids such as oleic acid are used in the preparation of injectables.

The injectable formulations can be sterilized, for example, by filtration through a bacteria-retaining filter, or by incorporating sterilizing agents in the form of sterile solid compositions which can be dissolved or dispersed in sterile water or other sterile injectable medium prior to use.

In order to prolong the effect of a compound of the present invention, it is often desirable to slow the absorption of the compound from subcutaneous or intramuscular injection. This can be accomplished by the use of a liquid suspension of crystalline or amorphous material with poor water solubility. The rate of absorption of the compound then depends upon its rate of dissolution, which in turn may depend upon crystal size and crystalline form. Alternatively, delayed absorption of a parenterally administered compound form is accomplished by dissolving or suspending the compound in an oil vehicle. Injectable depot forms are made by forming microencapsulated matrices of the compound in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

Compositions for rectal or vaginal administration are preferably suppositories which can be prepared by mixing the compounds of the present invention with suitable non-irritating excipients or carriers such as cocoa butter, polyethylene glycol or a suppository wax which are solid at ambient temperature but liquid at body temperature and therefore melt in the rectal or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid; b) binders such as, for example, carboxymethylcellulose, alginates, gelatin, polyvinylpyrrolidone, sucrose, and acacia; c) humectants such as glycerol; d) disintegrating agents such as agar-agar, calcium carbonate, potato or tapioca starch, alginic acid, certain silicates, and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite; and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polyethylene glycols and the like.

The active compounds can also be in micro-encapsulated form with one or more excipients as noted above. The solid dosage forms of tablets, dragees, capsules, pills, and granules can be prepared with coatings and shells such as enteric coatings, controlled release coatings and other coatings well known in the pharmaceutical formulating art. In such solid dosage forms, the active compound may be admixed with at least one inert diluent such as sucrose, lactose or starch. Such dosage forms may also comprise, as is normal practice, additional substances other than inert diluents, e.g., tableting lubricants and other tableting aids such as magnesium stearate and microcrystalline cellulose. In the case of capsules, tablets and pills, the dosage form may also comprise buffering agents. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

Dosage forms for topical or transdermal administration of a compound of the present invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulations, ear drops, and eye drops are also contemplated as being within the scope of the present invention. Additionally, the present invention contemplates the use of transdermal patches, which have the added advantage of providing controlled delivery of a compound to the body. Such dosage forms are prepared by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

The activity of the compounds used as glucocorticoid receptor modulators in the present invention may be assayed according to methods generally described in the art and in the examples herein.

The compounds of the present invention may be administered alone, or in combination with other pharmaceutically acceptable compounds.

When using the pharmaceutical composition, a safe and effective amount of the compound of the present invention is administered to a mammal (such as a human) in need of treatment, wherein the dosage at the time of administration is a pharmaceutically effective dosage, and for a human with a body weight of 60 kg, the daily dosage is usually 1 to 2000 mg, preferably 5 to 500 mg. Of course, the specific dose should also take into account factors such as the route of administration, the patient's health status, and the like, all of which are within the skill of a skilled physician.

### Preparation methods

### Method 1:

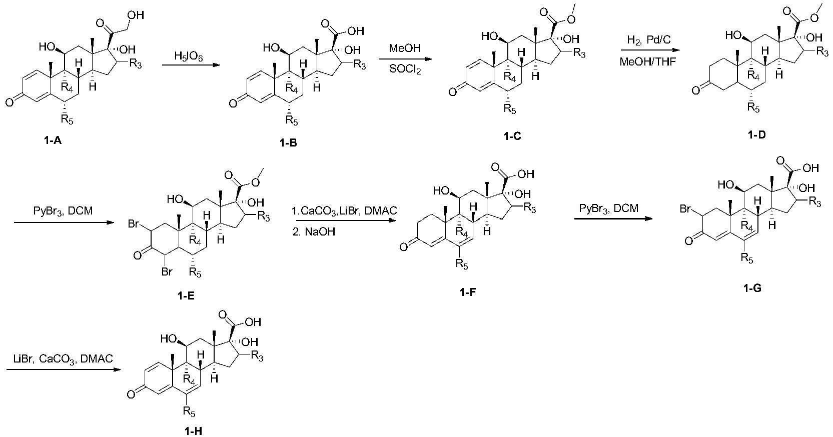

### Method 2:

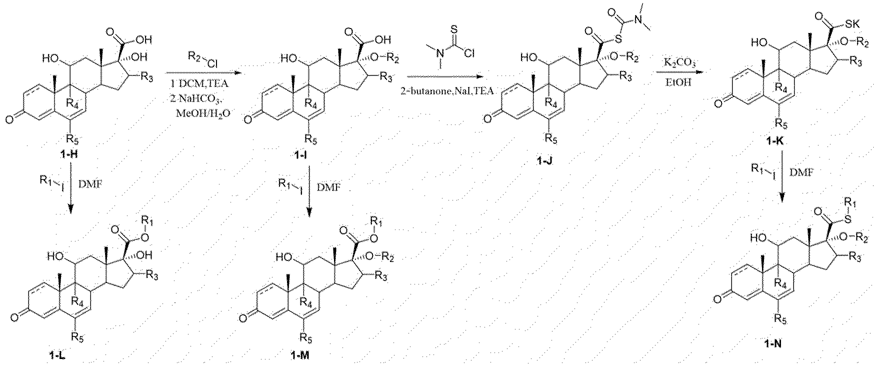

### Advantages of the Present Invention:

Compared with conventional and the latest glucocorticoid drugs, the glucocorticoid compounds of the present invention exhibit stronger glucocorticoid receptor affinity and stronger *in vivo* anti-inflammatory effects, and can achieve the same therapeutic effect at a lower dose, thereby minimizing side effects.

The present invention is further described below in conjunction with specific examples. It should be understood that these examples are only used to illustrate the present invention and not to limit the scope of the present invention. In the following examples, experimental methods without specific conditions noted are generally carried out according to conventional conditions or according to the conditions recommended by the manufacturer. Unless otherwise stated, percentages and parts are weight percentages and parts by weight.

### Example 1: Synthesis of Compound PA7200

### Step 1: Synthesis of Intermediate Compound 1-B

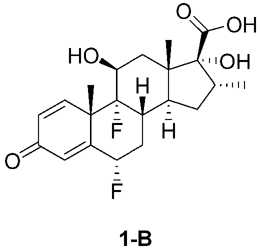

Under a nitrogen atmosphere, commercially available compound 1-A (50 g, 122 mmol) and anhydrous ethanol (150 mL) were charged into a 500 mL reaction flask. The system was cooled to 10-15 °C with stirring, and periodic acid (36.1 g, 159 mmol) was slowly added to the reaction system while controlling the temperature below 30 °C. After the addition was completed, stirring was continued at 25-30 °C for 3 hours. Monitored by LC-MS, compound 1-A had essentially disappeared. Drinking water (150 mL) was added to the reaction system and stirred for 1 hour. After filtration, the filter cake was washed with a mixture of ethanol and water (1:1). The solid was dried in an oven to obtain 48.1 g of compound 1-B, with a yield of 99.6%. LC-MS: [M+1]⁺ = 397.2.

NMR: ¹H NMR (400 MHz, DMSO-d₆) δ 12.49 (s, 1H), 7.29 (d, J = 10.4 Hz, 1H), 6.31 (dd, J = 10.2, 1.7 Hz, 1H), 6.12 (s, 1H), 5.65 (ddd, J = 48.7, 10.0, 6.8 Hz, 1H), 5.36 (d, J = 2.1 Hz, 1H), 4.72 (s, 1H), 4.16 (d, J = 9.4 Hz, 1H), 2.94-2.79 (m, 1H), 2.48-2.37 (m, 1H), 2.28-2.18 (m, 1H), 2.16-1.97 (m, 2H), 1.73-1.60 (m, 1H), 1.60-1.39 (m, 5H), 1.18-1.06 (m, 1H), 1.01 (s, 3H), 0.89 (d, J = 7.1 Hz, 3H).

### Step 2: Synthesis of Intermediate Compound 1-C

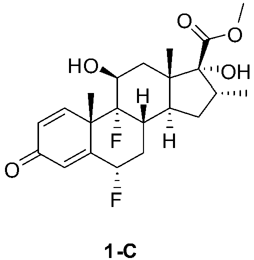

Under a nitrogen atmosphere, compound 1-B (48 g, 121 mmol) and methanol (190 mL) were charged into a 500 mL reaction flask. The system was cooled to 5-15 °C with stirring, and thionyl chloride (21.41 g, 182 mmol) was slowly added dropwise while controlling the temperature below 20 °C. After the dropwise addition was completed, the temperature was raised to 60 °C and reacted overnight. Monitored by LC-MS, compound 1-B had essentially disappeared. The organic solvent was spun to dryness. The obtained crude compound 1-C was slurried with a mixed solution of ethyl acetate (48 mL) and petroleum ether (144 mL) and stirred for 2 hours. After filtration, the solid was dried in an oven to obtain 48.7 g of compound 1-C, with a yield of 97.76%. LC-MS: [M+1]⁺ = 411.1.

NMR: ¹H NMR (400 MHz, DMSO-d₆) δ 7.34 (dd, J = 28.3, 10.2 Hz, 1H), 6.32 (dd, J = 10.1, 1.5 Hz, 1H), 6.14 (s, 1H), 5.66 (ddd, J = 48.8, 10.5, 7.0 Hz, 1H), 5.37 (t, J = 19.9 Hz, 1H), 5.04 (s, 1H), 4.17 (d, J = 10.1 Hz, 1H), 3.67 (s, 3H), 2.62-2.37 (m, 2H), 2.22 (dd, J = 20.8, 16.1 Hz, 1H), 2.20-1.95 (m, 2H), 1.70 (q, J = 11.8 Hz, 1H), 1.63-1.41 (m, 5H), 1.21-1.02 (m, 1H), 1.02-0.81 (m, 6H).

### Step 3: Synthesis of Intermediate Compound 1-D

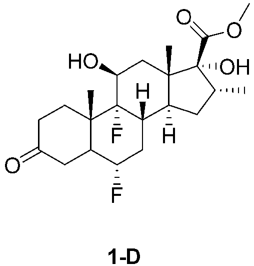

Compound 1-C (48.5 g, 118 mmol), methanol (170 mL) and tetrahydrofuran (72 mL) were charged into a 500 mL reaction flask. After stirring uniformly, 6.3 g of Pd/C (10%) was added, and the system was purged with hydrogen three times, followed by stirring at room temperature under a hydrogen atmosphere for 2 days. Monitored by LC-MS, compound 1-C and the intermediate state had essentially disappeared. The reaction solution was filtered through celite, and the filter cake was washed with tetrahydrofuran (72 mL). The filtrate was concentrated to dryness under reduced pressure, and the solid was dried in an oven to obtain 50 g of compound 1-D, with a yield of 98.61%. LC-MS: [M+H₂O]⁺ = 432.5.

NMR: ¹H NMR (400 MHz, DMSO-d₆) δ 5.14 (ddd, J = 17.8, 11.6, 5.4 Hz, 1H), 5.03-4.91 (m, 2H), 4.23 (s, 1H), 3.66 (s, 3H), 2.90 (dd, J = 12.5, 5.5 Hz, 1H), 2.66-2.55 (m, 2H), 2.35 (dd, J = 24.5, 13.7 Hz, 2H), 2.28-2.03 (m, 5H), 1.81 (d, J = 3.4 Hz, 1H), 1.73-1.58 (m, 3H), 1.52 (d, J = 13.9 Hz, 1H), 1.27 (s, 3H), 1.10 (dd, J = 15.0, 11.4 Hz, 1H), 0.98-0.88 (m, 6H).

### Step 4: Synthesis of Intermediate Compound 1-E

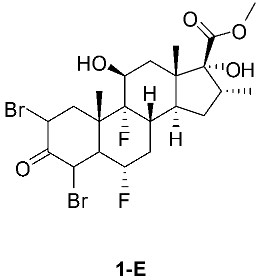

Under a nitrogen atmosphere, compound 1-D (49.5 g, 119 mmol) and dichloromethane (1500 mL) were charged into a 5 L reaction flask. The system was cooled to 0-10 °C with stirring, and pyridinium tribromide (41.3 g, 262 mmol) was added in portions. After the addition was completed, the reaction was carried out at room temperature for 3 hours. Monitored by LC-MS, compound 1-D had essentially disappeared. Drinking water (1500 mL) was added to the reaction system, and the organic phase was separated. The organic phase was washed twice with drinking water (1500 mL), washed with saturated brine (1500 mL), and dried over anhydrous sodium sulfate. After filtration, the organic phase was concentrated to dryness under reduced pressure, and the solid was dried in an oven to obtain 69.4 g of crude compound 1-E. LC-MS: [M+H₂O]⁺ = 589.9.

NMR: ¹H NMR (400 MHz, DMSO-d₆) δ 5.14 (ddd, J = 17.8, 11.6, 5.4 Hz, 1H), 5.03-4.91 (m, 2H), 4.23 (s, 1H), 3.67 (s, 3H), 2.90 (dd, J = 12.5, 5.5 Hz, 1H), 2.66-2.55 (m, 1H), 2.35 (dd, J = 24.5, 13.7 Hz, 1H), 2.20-2.07 (m, 5H), 1.84 (d, J = 3.4 Hz, 1H), 1.58 (m, 3H), 1.50 (d, J = 13.9 Hz, 1H), 1.33 (s, 3H), 1.20 (dd, J = 15.0, 11.4 Hz, 1H), 0.99-0.88 (m, 6H).

### Step 5: Synthesis of Intermediate Compound 1-F

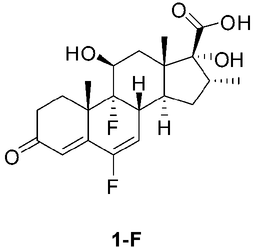

Under a nitrogen atmosphere, crude compound 1-E (69 g, 119 mmol) and DMAC (255 mL) were charged into a 500 mL reaction flask. After stirring uniformly, the system was heated to 45 °C, lithium bromide (20.67 g, 238 mmol) was added, and the mixture was stirred for 5 minutes. Then calcium carbonate (23.8 g, 238 mmol) was added, and the system was heated to 95 °C and reacted for another 3 hours. The system was cooled to room temperature, and 4 M aqueous sodium hydroxide solution (500 mL) was added to the reaction system, followed by reaction at room temperature for 3 hours. The pH was then adjusted to 1-2 with 2 mol/L hydrochloric acid solution, and ethyl acetate (500 mL) was added for extraction. The organic phase was separated, washed twice with drinking water (500 mL), once with saturated brine (500 mL), and dried over anhydrous sodium sulfate. After filtration, the organic phase was concentrated to dryness under reduced pressure, and the solid was dried in an oven to obtain 49.7 g of crude compound 1-F. The crude product (49.7 g) was added to acetonitrile (150 mL), stirred for 2 hours, filtered, and the filter cake was washed with acetonitrile (5 mL). The filter cake was added to a mixed solution of acetonitrile (100 mL) and methanol (100 mL), stirred for two hours, filtered, and the solid was dried in an oven to obtain 15.7 g of compound 1-F, with a two-step yield of 32.75%. LC-MS: [M+1]⁺ = 397.0.

NMR: ¹H NMR (400 MHz, DMSO-d₆) δ 12.41 (s, 1H), 5.87 (s, 1H), 5.64 (d, J = 15.1 Hz, 1H), 5.22 (d, J = 1.9 Hz, 1H), 4.13 (s, 1H), 3.18-2.82 (m, 2H), 2.78-2.59 (m, 1H), 2.37 (dd, J = 19.8, 12.5 Hz, 2H), 2.24 (td, J = 14.0, 4.7 Hz, 1H), 2.17-2.02 (m, 2H), 1.76 (q, J = 11.4 Hz, 1H), 1.65-1.41 (m, 5H), 1.36-1.18 (m, 1H), 1.06 (d, J = 6.3 Hz, 3H), 0.92 (d, J = 7.1 Hz, 3H).

### Step 6: Synthesis of Intermediate Compound 1-G

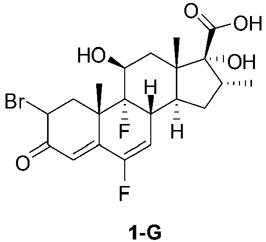

Under a nitrogen atmosphere, compound 1-F (900 mg, 2.27 mmol) and dichloromethane (30 mL) were charged into a 100 mL flask. After stirring uniformly, the system was cooled to 10 °C, and pyridinium tribromide (376 mg, 2.38 mmol) was added in portions. After the addition was completed, the reaction was continued for 6 hours. Monitored by TLC, compound 1-F had essentially disappeared. Water (50 mL) was added to the reaction system, and the organic phase was separated. The organic phase was washed twice with drinking water (50 mL), once with saturated brine (50 mL), and dried over anhydrous sodium sulfate. After filtration, the organic phase was concentrated to dryness under reduced pressure, and the solid was dried in an oven to obtain 1.1 g of crude compound 1-G. LC-MS: [M+1]⁺ = 474.9.

### Step 7: Synthesis of Intermediate Compound 1-H

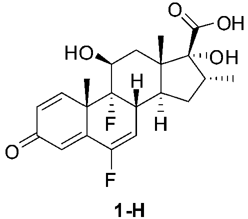

Under a nitrogen atmosphere, crude compound 1-G (1.1 g, 2.31 mmol) and DMAC (20 mL) were charged into a 100 mL reaction flask. After stirring uniformly, the system was heated to 45 °C, lithium bromide (400 mg, 4.62 mmol) was added, and the mixture was stirred for 5 minutes. Then calcium carbonate (462 mg, 4.62 mmol) was added, and the system was heated to 95 °C and reacted for another 3 hours. The system was cooled to room temperature, and the pH was adjusted to 1-2 with 2 mol/L hydrochloric acid solution. Ethyl acetate (50 mL) was added to the reaction system for extraction, and the organic phase was separated. The organic phase was washed twice with drinking water (50 mL), once with saturated brine (50 mL), dried over anhydrous sodium sulfate, filtered, and the organic phase was concentrated to dryness under reduced pressure. The solid was dried in an oven to obtain 890 mg of the compound, with a two-step yield of 97%. LC-MS: [M+1]⁺ = 395.0.

NMR: ¹H NMR (400 MHz, DMSO-d₆) δ 14.71-9.60 (m, 1H), 7.42 (d, J = 10.2 Hz, 1H), 6.35 (d, J = 10.1 Hz, 1H), 6.22 (s, 1H), 5.64 (d, J = 14.9 Hz, 1H), 5.56 (s, 1H), 4.25 (s, 1H), 3.19 (s, 1H), 3.09 (dd, J = 24.2, 9.2 Hz, 1H), 2.91 (d, J = 6.9 Hz, 1H), 2.34 (dd, J = 20.3, 12.0 Hz, 1H), 2.18-2.06 (m, 1H), 1.78 (q, J = 11.5 Hz, 1H), 1.64-1.50 (m, 4H), 1.26 (dd, J = 14.1, 6.1 Hz, 1H), 1.07 (s, 3H), 0.91 (d, J = 7.1 Hz, 3H).

### Step 8: Synthesis of Compound 1-I:

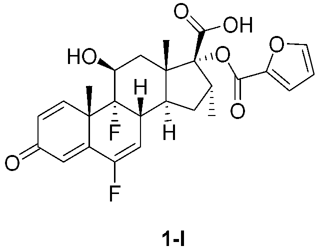

Under a nitrogen atmosphere, compound 1-H (445 mg, 1.13 mmol), DCM (5 mL) and triethylamine (303 mg, 2.99 mmol) were charged into a 50 mL reaction flask. After stirring uniformly, the system was cooled to 5 °C, and furoyl chloride (366 mg, 2.82 mmol) was slowly added dropwise. After the dropwise addition was completed, the reaction was carried out at room temperature for 1 hour. Monitored by TLC, the starting material had disappeared and converted to an intermediate state. The organic solvent was concentrated to dryness under reduced pressure. The crude product was added to methanol (8.8 mL), sodium bicarbonate (230 mg, 2.74 mmol) and water (2.3 mL) with stirring. The system was heated to 48 °C for further reaction for 10 hours. Monitored by TLC, all intermediate states had converted to the product. The pH was adjusted to 1-2 with 1 mol/L hydrochloric acid, filtered, and the filter cake was washed with drinking water (5 mL). The solid was dried in an oven to obtain 490 mg of compound 1-I, with a yield of 88.9%. LC-MS: [M+1]⁺ = 489.0.

### Step 9: Synthesis of Intermediate Compound 1-J

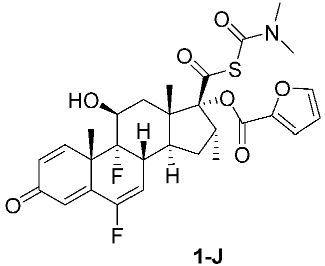

Under a nitrogen atmosphere, compound 1-I (490 mg, 1 mmol), sodium iodide (179 mg, 1.2 mmol), triethylamine (202 mg, 2 mmol) and 2-butanone (10 mL) were charged into a 50 mL reaction flask. After stirring uniformly, the system was cooled to 0-5 °C, and dimethylthiocarbamoyl chloride (185 mg, 1.5 mmol) was added, followed by reaction at room temperature for 16 hours. Monitored by TLC, the starting material had disappeared. Saturated brine (50 mL) was added to the reaction system to quench the reaction, and then ethyl acetate (50 mL) was added for extraction. The organic phase was separated, dried over anhydrous sodium sulfate, filtered, and the organic phase was concentrated to dryness under reduced pressure. The solid was dried in an oven to obtain 507 mg of compound 1-J, with a yield of 87.8%.

### Step 10: Synthesis of Intermediate Compound 1-K

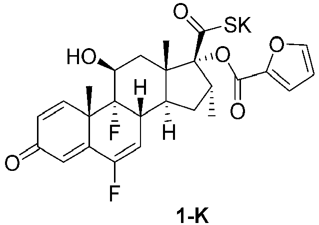

Under a nitrogen atmosphere, compound 1-J (490 mg, 0.85 mmol), 100-mesh potassium carbonate (351 mg, 2.54 mmol) and ethanol (5 mL) were charged into a 50 mL reaction flask. After stirring uniformly, the reaction was carried out at room temperature for 16 hours. Monitored by TLC, the starting material had essentially disappeared. After filtration, the filter cake was washed with water (5 mL), and the solid was dried in an oven to obtain 800 mg of compound 1-K. The crude product was used directly in the next step without purification. LC-MS: [M-K]⁻ = 503.0.

### Step 11: Synthesis of Compound PA7200

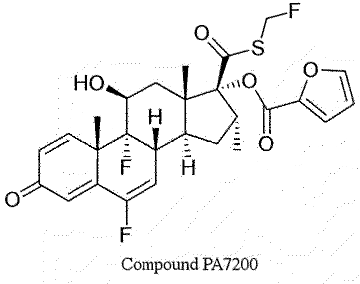

Under a nitrogen atmosphere, crude compound 1-K (400 mg, theoretical value: 0.43 mmol) and DMF (5 mL) were charged into a 50 mL reaction flask. The system was cooled to 0-5 °C, and fluoroiodomethane (101 mg, 0.64 mmol) was slowly added. After stirring uniformly, the reaction was carried out at room temperature for 2 hours. Monitored by TLC, the starting material had essentially disappeared. Drinking water (50 mL) was added to the reaction system to quench the reaction, and then ethyl acetate (50 mL) was added for extraction. The organic phase was separated, washed once with saturated brine (50 mL), and dried over anhydrous sodium sulfate. The organic phase was concentrated to dryness under reduced pressure, purified by reverse-phase column chromatography, and lyophilized to obtain 90 mg of compound PA7200, with a two-step yield of 24.3% and an HPLC purity of 95.52%. LC-MS: [M+1]⁺ = 537.1.

NMR: ¹H NMR (400 MHz, DMSO-d₆) δ 8.04 (s, 1H), 7.43 (d, J = 9.9 Hz, 1H), 7.24 (d, J = 3.4 Hz, 1H), 6.73 (dd, J = 3.5, 1.7 Hz, 1H), 6.39 (d, J = 10.2 Hz, 1H), 6.26 (s, 1H), 6.05 (s, 1H), 5.92 (s, 1H), 5.86 (d, J = 2.4 Hz, 1H), 5.71 (d, J = 15.0 Hz, 1H), 4.39 (s, 1H), 3.79-3.51 (m, 3H), 2.30 (d, J = 14.3 Hz, 1H), 2.06 (dd, J = 21.9, 10.0 Hz, 1H), 1.92 (d, J = 13.9 Hz, 1H), 1.55 (s, 3H), 1.50 (s, 1H), 1.13 (s, 3H), 0.98 (d, J = 7.1 Hz, 3H).

### Example 2: Synthesis of Compound PA7201

### Step 1: Synthesis of Intermediate Compound 1-L

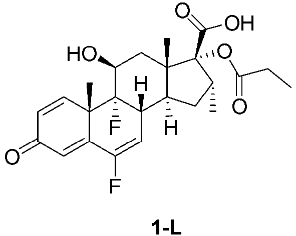

Under a nitrogen atmosphere, compound 1-H (445 mg, 1.13 mmol), DCM (5 mL) and triethylamine (303 mg, 2.99 mmol) were charged into a 50 mL reaction flask. After stirring uniformly, the system was cooled to 5 °C, and propionyl chloride (260 mg, 2.82 mmol) was slowly added dropwise. After the dropwise addition was completed, the mixture was stirred at room temperature for 1 hour. Monitored by TLC, the starting material had disappeared and converted to an intermediate state. The organic solvent was concentrated to dryness under reduced pressure. The crude product was added to methanol (8.8 mL), sodium bicarbonate (230 mg, 2.74 mmol) and water (2.3 mL) with stirring. The system was heated to 48 °C for further reaction for 10 hours. Monitored by TLC, all intermediate states had converted to the product. The pH was adjusted to 1-2 with 1 mol/L hydrochloric acid, filtered, and the filter cake was washed with drinking water (5 mL). The solid was dried in an oven to obtain 500 mg of compound 1-L, with a yield of 98.1%. LC-MS: [M+1]⁺ = 451.0.

### Step 2: Synthesis of Intermediate Compound 1-M

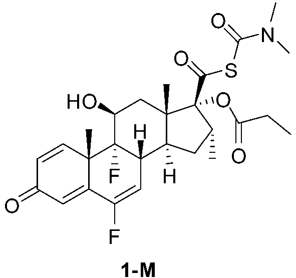

Under a nitrogen atmosphere, compound 1-L (500 mg, 1.1 mmol), sodium iodide (198 mg, 1.3 mmol), triethylamine (224 mg, 2.2 mmol) and 2-butanone (5 mL) were charged into a 50 mL reaction flask. After stirring uniformly, the system was cooled to 0-5 °C, and dimethylthiocarbamoyl chloride (185 mg, 1.5 mmol) was added, followed by reaction at room temperature for 16 hours. Monitored by TLC, the starting material had disappeared. Saturated brine (50 mL) was added to the reaction system to quench the reaction, and then ethyl acetate (50 mL) was added for extraction. The organic phase was separated, dried over anhydrous sodium sulfate, filtered, and the organic phase was concentrated to dryness under reduced pressure. The solid was dried in an oven to obtain 567 mg of compound 1-M, with a yield of 95%. LC-MS: [M+1]⁺ = 538.0.

### Step 3: Synthesis of Intermediate Compound 1-N:

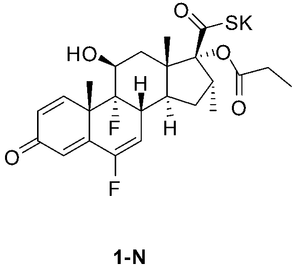

Under a nitrogen atmosphere, compound 1-M (567 mg, 1.1 mmol), 100-mesh potassium carbonate (456 mg, 3.3 mmol) and ethanol (5 mL) were charged into a 50 mL reaction flask. After stirring uniformly, the reaction was carried out at room temperature for 16 hours. Monitored by TLC, the starting material had essentially disappeared. After filtration, the filter cake was washed with anhydrous ethanol (5 mL), and the solid was dried in an oven to obtain 917 mg of compound 1-N. The crude product was used directly in the next step without purification. LC-MS: [M-K]⁻ = 465.0.

### Step 4: Synthesis of Compound PA7201

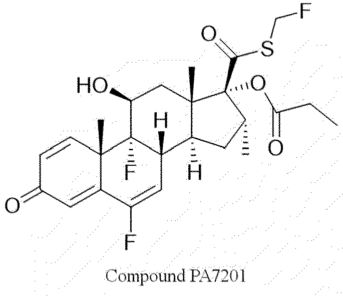

Under a nitrogen atmosphere, crude compound 1-N (917 mg, theoretical value: 1.1 mmol) and DMF (5 mL) were charged into a 50 mL reaction flask. The system was cooled to 0-5 °C, and fluoroiodomethane (264 mg, 1.65 mmol) was slowly added. After stirring uniformly, the reaction was carried out at room temperature for 2 hours. Monitored by TLC, the starting material had essentially disappeared. Drinking water (50 mL) was added to the reaction system to quench the reaction, and then ethyl acetate (50 mL) was added for extraction. The organic phase was separated, washed once with saturated brine (50 mL), and dried over anhydrous sodium sulfate. The organic phase was concentrated to dryness under reduced pressure, purified by reverse-phase column chromatography, and lyophilized to obtain 5 mg of compound PA7201, with an HPLC purity of 94.4% and a two-step yield of 0.55%. LC-MS: [M+1]⁺ = 499.1.

NMR: ¹H NMR (400 MHz, DMSO-d₆) δ 7.41 (d, J = 10.2 Hz, 1H), 6.37 (dd, J = 10.2, 1.7 Hz, 1H), 6.24 (s, 1H), 6.02 (s, 1H), 5.90 (s, 1H), 5.81 (d, J = 2.3 Hz, 1H), 5.68 (d, J = 14.2 Hz, 1H), 4.34 (s, 1H), 3.67-3.50 (m, 2H), 3.18 (d, J = 14.5 Hz, 1H), 2.44-2.29 (m, 3H), 2.20 (d, J = 14.0 Hz, 1H), 2.00 (d, J = 12.1 Hz, 1H), 1.88 (d, J = 14.1 Hz, 1H), 1.53 (s, 3H), 1.45 (t, J = 10.3 Hz, 1H), 1.04 (dd, J = 13.6, 6.1 Hz, 7H), 0.91 (dd, J = 19.0, 7.3 Hz, 4H).

### Example 3: Synthesis of Compound PA7202

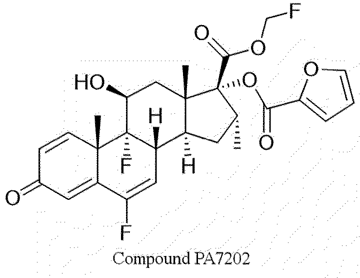

Under a nitrogen atmosphere, compound 1-I (160 mg, 0.33 mmol), potassium carbonate (135 mg, 0.97 mmol) and DMF (10 mL) were charged into a 100 mL reaction flask. The system was cooled to 0-5 °C, and fluoroiodomethane (78 mg, 0.49 mmol) was slowly added. After stirring uniformly, the reaction was carried out at room temperature for 2 hours. Monitored by TLC, the starting material had essentially disappeared. Drinking water (50 mL) was added to the reaction system to quench the reaction, and then ethyl acetate (50 mL) was added for extraction. The organic phase was separated, washed once with saturated brine (50 mL), and dried over anhydrous sodium sulfate. The organic phase was concentrated to dryness under reduced pressure, purified by reverse-phase column chromatography, and lyophilized to obtain 106 mg of compound PA7202, with an HPLC purity of 99.59% and a yield of 58.8%. LC-MS: [M+1]⁺ = 521.5.

NMR: ¹H NMR (400 MHz, DMSO-d₆) δ 8.01 (dd, J = 1.7, 0.8 Hz, 1H), 7.40 (d, J = 8.4 Hz, 1H), 7.22 (dd, J = 3.5, 0.7 Hz, 1H), 6.70 (dd, J = 3.6, 1.7 Hz, 1H), 6.36 (dd, J = 10.2, 1.9 Hz, 1H), 6.24 (s, 1H), 5.79 (ddd, J = 56.0, 40.0, 27.1 Hz, 4H), 4.33 (s, 1H), 3.21 (d, J = 44.4 Hz, 3H), 2.24 (d, J = 13.8 Hz, 1H), 2.06-1.93 (m, 1H), 1.75 (d, J = 14.1 Hz, 1H), 1.54 (s, 3H), 1.45 (s, 1H), 1.10 (s, 3H), 0.92 (d, J = 7.2 Hz, 3H).

### Example 4: Synthesis of Compound PA7203

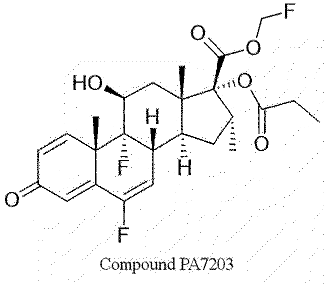

Under a nitrogen atmosphere, compound 1-L (144 mg, 0.32 mmol), potassium carbonate (132 mg, 0.96 mmol) and DMF (10 mL) were charged into a 100 mL reaction flask. Fluoroiodomethane (76 mg, 0.47 mmol) was slowly added. After stirring uniformly, the reaction was carried out at room temperature for 2 hours. Monitored by TLC, the starting material had essentially disappeared. Drinking water (50 mL) was added to the reaction system to quench the reaction, and then ethyl acetate (50 mL) was added for extraction. The organic phase was separated, washed once with saturated brine (50 mL), and dried over anhydrous sodium sulfate. The organic phase was concentrated to dryness under reduced pressure, purified by reverse-phase column chromatography, and lyophilized to obtain 60 mg of compound PA7203, with a yield of 42.3% and an HPLC purity of 98.02%. LC-MS: [M+1]⁺ = 483.7.

NMR: ¹H NMR (400 MHz, DMSO-d₆) δ 7.38 (d, J = 10.3 Hz, 1H), 6.35 (dd, J = 10.2, 1.9 Hz, 1H), 6.21 (s, 1H), 5.93-5.63 (m, 4H), 4.29 (s, 1H), 3.24 (s, 3H), 2.37-2.35 (m, 2H), 2.14 (d, J = 14.0 Hz, 1H), 1.94 (d, J = 11.9 Hz, 1H), 1.70 (d, J = 14.3 Hz, 1H), 1.52 (s, 3H), 1.39 (s, 1H), 1.05 (s, 3H), 1.01 (t, J = 7.5 Hz, 3H), 0.88 (d, J = 7.1 Hz, 3H).

### Example 5: Synthesis of Compound PA7204

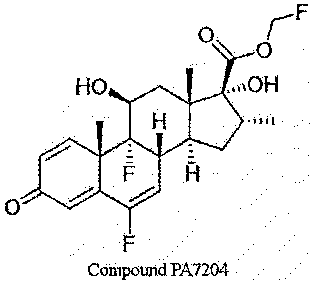

Under a nitrogen atmosphere, compound 1-H (130 mg, 0.33 mmol), potassium carbonate (136 mg, 0.98 mmol) and DMF (10 mL) were charged into a 100 mL reaction flask. The system was cooled to 0-5 °C, and fluoroiodomethane (79 mg, 0.49 mmol) was slowly added. After stirring uniformly, the reaction was carried out at room temperature for 2 hours. Monitored by TLC, the starting material had essentially disappeared. Drinking water (50 mL) was added to the reaction system to quench the reaction, and then ethyl acetate (50 mL) was added for extraction. The organic phase was separated, washed once with saturated brine (50 mL), and dried over anhydrous sodium sulfate. The organic phase was concentrated to dryness under reduced pressure, purified by reverse-phase column chromatography, and lyophilized to obtain 72 mg of compound PA7204, with an HPLC purity of 99.53% and a yield of 51.2%. LC-MS: [M+1]⁺ = 427.4.

NMR: ¹H NMR (400 MHz, DMSO-d₆) δ 7.39 (dd, J = 10.2, 1.8 Hz, 1H), 6.34 (dd, J = 10.2, 1.9 Hz, 1H), 6.20 (s, 1H), 5.98-5.57 (m, 4H), 5.35 (s, 1H), 4.24 (s, 1H), 3.08 (dd, J = 25.4, 8.0 Hz, 1H), 2.99-2.84 (m, 1H), 2.35 (dd, J = 15.8, 7.0 Hz, 1H), 2.15 (d, J = 13.9 Hz, 1H), 1.87-1.73 (m, 1H), 1.60 (d, J = 14.0 Hz, 1H), 1.51 (s, 3H), 1.27 (ddd, J = 12.3, 8.1, 3.8 Hz, 1H), 1.01 (s, 3H), 0.92 (d, J = 7.2 Hz, 3H).

### Example 6: Synthesis of Compound PA7100

### Step 1: Synthesis of Intermediate Compound 1-O

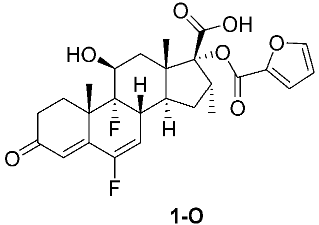

Under a nitrogen atmosphere, compound 1-F (1.0 g, 2.52 mmol), DCM (7 mL) and triethylamine (663 mg, 6.55 mmol) were charged into a 50 mL reaction flask. After stirring uniformly, the system was cooled to 0-5 °C, furoyl chloride (823 mg, 6.31 mmol) was added, and the mixture was stirred at room temperature for 1 hour. Monitored by TLC, the starting material had disappeared and converted to an intermediate state. The organic solvent was concentrated to dryness under reduced pressure. The crude product was added to methanol (20 mL), sodium bicarbonate (0.5 g, 6.23 mmol) and water (5.0 mL) with stirring. The system was heated to 48 °C for further reaction for 10 hours. Monitored by TLC, all intermediate states had converted to the product. The pH was adjusted to 1-2 with 1 mol/L hydrochloric acid, filtered, and the filter cake was washed with drinking water (5 mL). The solid was dried in an oven to obtain 1023 mg of compound 1-O, with a yield of 82.6%. LC-MS: [M+1]⁺ = 491.0.

NMR: ¹H NMR (400 MHz, DMSO-d₆) δ 12.92 (s, 1H), 8.02 (s, 1H), 7.17 (d, J = 3.2 Hz, 1H), 6.73 (s, 1H), 5.90 (s, 1H), 5.72 (d, J = 14.7 Hz, 1H), 5.41 (s, 1H), 4.20 (s, 1H), 3.24-2.98 (m, 2H), 2.68 (d, J = 13.8 Hz, 1H), 2.39-2.17 (m, 3H), 2.10 (s, 1H), 1.95 (d, J = 11.3 Hz, 1H), 1.73 (d, J = 15.4 Hz, 1H), 1.49 (s, 3H), 1.43 (s, 1H), 1.20 (t, J = 7.2 Hz, 1H), 1.11 (s, 3H), 0.94 (d, J = 7.0 Hz, 3H).

### Step 2: Synthesis of Intermediate Compound 1-P

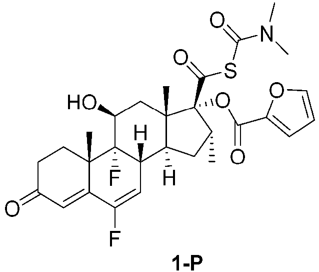

Under a nitrogen atmosphere, compound 1-O (1.0 g, 2.04 mmol), sodium iodide (366 mg, 2.24 mmol), triethylamine (412 mg, 4.08 mmol) and 2-butanone (10 mL) were charged into a 50 mL reaction flask. After stirring uniformly, the system was cooled to 0-5 °C, dimethylthiocarbamoyl chloride (378 mg, 3.05 mmol) was added, and the reaction was carried out at room temperature for 16 hours. Monitored by TLC, the starting material had disappeared. Saturated brine (50 mL) was added to the reaction system to quench the reaction, and then ethyl acetate (50 mL) was added for extraction. The organic phase was separated, dried over anhydrous sodium sulfate, filtered, and the organic phase was concentrated to dryness under reduced pressure. The solid was dried in an oven to obtain 931 mg of compound 1-P, with a yield of 79.06%.

NMR: ¹H NMR (400 MHz, DMSO-d₆) δ 8.08 (d, J = 0.9 Hz, 1H), 7.27 (d, J = 3.5 Hz, 1H), 6.77 (dd, J = 3.5, 1.7 Hz, 1H), 5.92 (s, 1H), 5.73 (d, J = 15.0 Hz, 1H), 5.58 (s, 1H), 4.26 (s, 1H), 3.46 (t, J = 5.8 Hz, 2H), 3.16 (d, J = 23.5 Hz, 4H), 3.01 (s, 4H), 2.77-2.64 (m, 1H), 2.35-2.21 (m, 2H), 2.17-1.98 (m, 2H), 1.92 (d, J = 13.8 Hz, 1H), 1.51 (s, 4H), 1.25-1.19 (m, 3H), 0.99 (d, J = 7.0 Hz, 3H).

### Step 3: Synthesis of Compound 1-Q

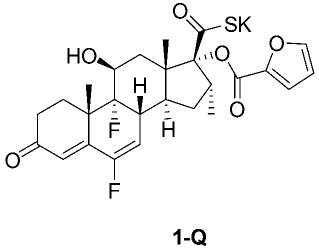

Under a nitrogen atmosphere, compound 1-P (900 mg, 1.56 mmol), 100-mesh potassium carbonate (646 mg, 4.671 mmol) and ethanol (5 mL) were charged into a 50 mL reaction flask. After stirring uniformly, the reaction was carried out at room temperature for 16 hours. Monitored by TLC, the starting material had essentially disappeared. After filtration, the filter cake was washed with anhydrous ethanol (5 mL), and the solid was dried in an oven to obtain 1.7 g of compound 1-Q. The crude product was used directly in the next step without purification. LC-MS: [M-K]⁻ = 507.0.

### Step 4: Synthesis of Compound PA7100

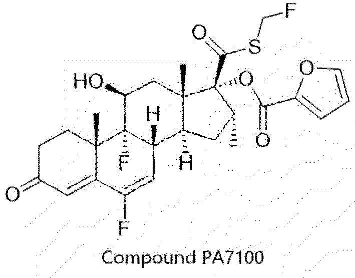

Under a nitrogen atmosphere, crude compound 1-Q (1.0 g, theoretical value: 0.92 mmol) and DMF (10 mL) were charged into a 50 mL reaction flask. The system was cooled to 0-5 °C, and fluoroiodomethane (219 mg, 1.37 mmol) was slowly added. After stirring uniformly, the reaction was carried out at room temperature for 2 hours. Monitored by TLC, the starting material had essentially disappeared. Drinking water (50 mL) was added to the reaction system to quench the reaction, and then ethyl acetate (50 mL) was added for extraction. The organic phase was separated, washed once with saturated brine (50 mL), and dried over anhydrous sodium sulfate. The organic phase was concentrated to dryness under reduced pressure, purified by reverse-phase column chromatography, and lyophilized to obtain 80 mg of compound PA7100, with an HPLC purity of 92.11% and a two-step yield of 80.89%. LC-MS: [M+1]⁺ = 539.1.

NMR: ¹H NMR (400 MHz, DMSO-d₆) δ 8.05 (d, J = 5.2 Hz, 1H), 7.42 (s, 1H), 7.25 (d, J = 3.4 Hz, 1H), 6.80-6.70 (m, 1H), 6.38 (dt, J = 8.8, 4.4 Hz, 1H), 6.25 (d, J = 9.8 Hz, 1H), 6.02 (d, J = 27.8 Hz, 1H), 5.92 (d, J = 11.7 Hz, 1H), 5.82 (t, J = 10.9 Hz, 1H), 5.77-5.64 (m, 1H), 4.39 (s, 1H), 3.43 (d, J = 7.1 Hz, 1H), 3.30-3.16 (m, 1H), 2.39-2.26 (m, 1H), 2.13-1.98 (m, 1H), 1.93 (d, J = 13.7 Hz, 1H), 1.59-1.45 (m, 5H), 1.13 (s, 3H), 1.04-0.94 (m, 3H).

### Example 7: Synthesis of Compound PA7101

### Step 1: Synthesis of Intermediate Compound 1-R

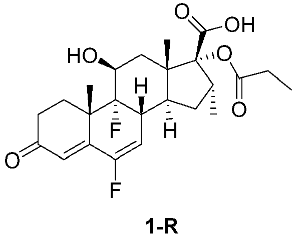

Under a nitrogen atmosphere, compound 1-F (1.0 g, 2.52 mmol), DCM (7 mL) and triethylamine (663 mg, 6.55 mmol) were charged into a 50 mL reaction flask. After stirring uniformly, the system was cooled to 0-5°C, propionyl chloride (583 mg, 6.31 mmol) was slowly added, and the mixture was stirred at room temperature for 1 hour. Monitored by TLC, the starting material had disappeared and converted to an intermediate state. The organic solvent was concentrated to dryness under reduced pressure. The crude product was added to methanol (20 mL), sodium bicarbonate (0.5 g, 6.23 mmol) and water (5 mL) with stirring. The system was heated to 48 °C for further reaction for 10 hours. Monitored by TLC, all intermediate states had converted to the product. The pH was adjusted to 1-2 with 1 mol/L hydrochloric acid, filtered, and the filter cake was washed with drinking water (5 mL). The solid was dried in an oven to obtain 930 mg of compound 1-R, with a yield of 81.4%. [M+1]⁺ = 453.1.

NMR: ¹H NMR (400 MHz, DMSO-d₆) δ 12.61 (s, 1H), 5.88 (s, 1H), 5.69 (d, J = 14.4 Hz, 1H), 5.37 (d, J = 2.3 Hz, 1H), 4.16 (s, 1H), 3.29-3.17 (m, 1H), 3.15-3.00 (m, 1H), 2.74-2.61 (m, 1H), 2.45-2.31 (m, 4H), 2.29-2.19 (m, 1H), 2.11 (t, J = 14.6 Hz, 2H), 1.89 (q, J = 11.7 Hz, 1H), 1.69 (d, J = 14.1 Hz, 1H), 1.48 (s, 3H), 1.42-1.31 (m, 1H), 1.10-1.01 (m, 6H), 0.89 (d, J = 7.1 Hz, 3H).

### Step 2: Synthesis of Intermediate Compound 1-S

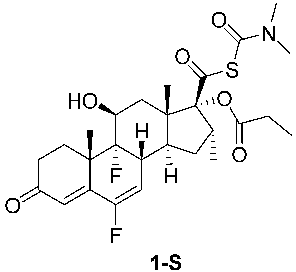

Under a nitrogen atmosphere, compound 1-R (900 mg, 1.99 mmol), sodium iodide (359 mg, 2.4 mmol), triethylamine (404 mg, 4.0 mmol) and 2-butanone (5 mL) were charged into a 50 mL reaction flask. After stirring uniformly, the system was cooled to 0-5°C, dimethylthiocarbamoyl chloride (369 mg, 3.0 mmol) was added, and the reaction was carried out at room temperature for 16 hours. Monitored by TLC, the starting material had disappeared. Saturated brine (50 mL) was added to the reaction system to quench the reaction, and then ethyl acetate (50 mL) was added for extraction. The organic phase was separated, dried over anhydrous sodium sulfate, filtered, and the organic phase was concentrated to dryness under reduced pressure. The solid was dried in an oven to obtain 891 mg of compound 1-S, with a yield of 83.02%.

### Step 3: Synthesis of Intermediate Compound 1-T

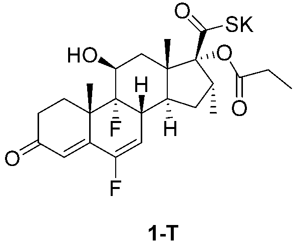

Under a nitrogen atmosphere, compound 1-S (891 mg, 1.65 mmol), 100-mesh potassium carbonate (684 mg, 4.95 mmol) and ethanol (5 mL) were charged into a 50 mL reaction flask. After stirring uniformly, the reaction was carried out at room temperature for 16 hours. Monitored by TLC, the starting material had essentially disappeared. After filtration, the filter cake was washed with anhydrous ethanol (5 mL), and the solid was dried in an oven to obtain 1.5 g of compound 1-T. The crude product was used directly in the next step without purification. LC-MS: [M-K]⁻ = 467.1.

### Step 4: Synthesis of Compound PA7101

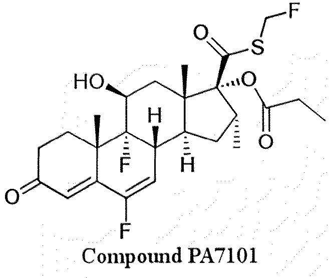

Under a nitrogen atmosphere, crude compound 1-T (750 mg, theoretical value: 0.83 mmol) and DMF (5 mL) were charged into a 50 mL reaction flask. The system was cooled to 0-5 °C, and fluoroiodomethane (198 mg, 1.24 mmol) was slowly added. After stirring uniformly, the reaction was carried out at room temperature for 2 hours. Monitored by TLC, the starting material had essentially disappeared. Drinking water (50 mL) was added to the reaction system to quench the reaction, and then ethyl acetate (50 mL) was added for extraction. The organic phase was separated, washed once with saturated brine (50 mL), and dried over anhydrous sodium sulfate. The organic phase was concentrated to dryness under reduced pressure, purified by reverse-phase column chromatography, and lyophilized to obtain 46 mg of compound PA7101, with an HPLC purity of 95.64% and a two-step yield of 6.2%. LC-MS: [M+1]⁺ = 501.3.

NMR: ¹H NMR (400 MHz, DMSO-d₆) δ 6.02 (s, 1H), 5.89 (d, J = 4.6 Hz, 2H), 5.70 (d, J = 14.8 Hz, 1H), 5.50 (d, J = 3.0 Hz, 1H), 4.17 (d, J = 25.2 Hz, 1H), 3.36 (s, 1H), 3.10 (ddd, J = 16.4, 11.5, 4.0 Hz, 1H), 2.77-2.62 (m, 1H), 2.47-2.31 (m, 4H), 2.32-2.16 (m, 2H), 2.14-2.02 (m, 1H), 1.98 (q, J = 11.8 Hz, 1H), 1.85 (d, J = 13.8 Hz, 1H), 1.54-1.38 (m, 4H), 1.11-1.00 (m, 6H), 0.92 (dd, J = 18.8, 7.0 Hz, 3H).

### Example 8: Synthesis of Compound PA7102

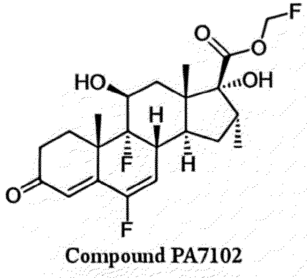

Under a nitrogen atmosphere, compound 1-F (100 mg, 0.25 mmol), 100-mesh potassium carbonate (104 mg, 0.76 mmol) and DMF (5 mL) were charged into a 50 mL reaction flask. The system was cooled to 0-5°C, and fluoroiodomethane (60 mg, 0.38 mmol) was slowly added. After stirring uniformly, the reaction was carried out at room temperature for 2 hours. Monitored by TLC, the starting material had essentially disappeared. Drinking water (50 mL) was added to the reaction system to quench the reaction, and then ethyl acetate (50 mL) was added for extraction. The organic phase was separated, washed once with saturated brine (50 mL), and dried over anhydrous sodium sulfate. The organic phase was concentrated to dryness under reduced pressure, purified by reverse-phase column chromatography, and lyophilized to obtain 53 mg of compound PA7102, with an HPLC purity of 96.26% and a yield of 49.03%. LC-MS: [M+1]⁺ = 429.7.

NMR: ¹H NMR (500 MHz, DMSO-d₆) δ 5.92 (d, J = 2.0 Hz, 1H), 5.85 (s, 1H), 5.80 (dd, J = 11.1, 2.1 Hz, 1H), 5.69 (d, J = 2.1 Hz, 1H), 5.63 (d, J = 14.5 Hz, 1H), 5.31 (d, J = 20.6 Hz, 2H), 4.11 (d, J = 5.2 Hz, 1H), 3.08-2.90 (m, 2H), 2.71-2.59 (m, 1H), 2.44-2.32 (m, 2H), 2.26-2.11 (m, 2H), 2.06 (d, J = 7.3 Hz, 1H), 1.78 (q, J = 11.2 Hz, 1H), 1.58-1.50 (m, 1H), 1.45 (s, 3H), 1.28 (ddd, J = 12.3, 8.1, 4.2 Hz, 1H), 1.04-0.98 (m, 3H), 0.92 (d, J = 7.2 Hz, 3H).

### Example 9: Evaluation Test of Glucocorticoid Receptor (hereinafter referred to as "GR") Binding Activity

Dexamethasone is a glucocorticoid first synthesized in 1957. It has pharmacological effects such as anti-inflammation, anti-endotoxin, immunosuppression, anti-shock and enhancement of stress response, and is included in the World Health Organization Model List of Essential Medicines as one of the essential drugs for the basic public health system. Its structural formula is shown below.

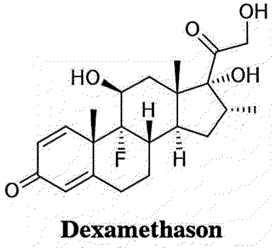

Fluticasone furoate is a state-of-the-art novel glucocorticoid developed by GlaxoSmithKline. It was first marketed in the UK in 1993 for the treatment of asthma, allergic rhinitis, chronic obstructive pulmonary disease, atopic dermatitis, etc. Compared with other glucocorticoids, it exhibits stronger receptor affinity and more potent anti-inflammatory effects. Its structural formula is shown below.

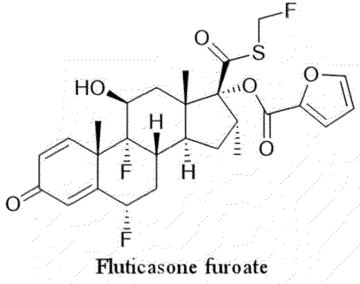

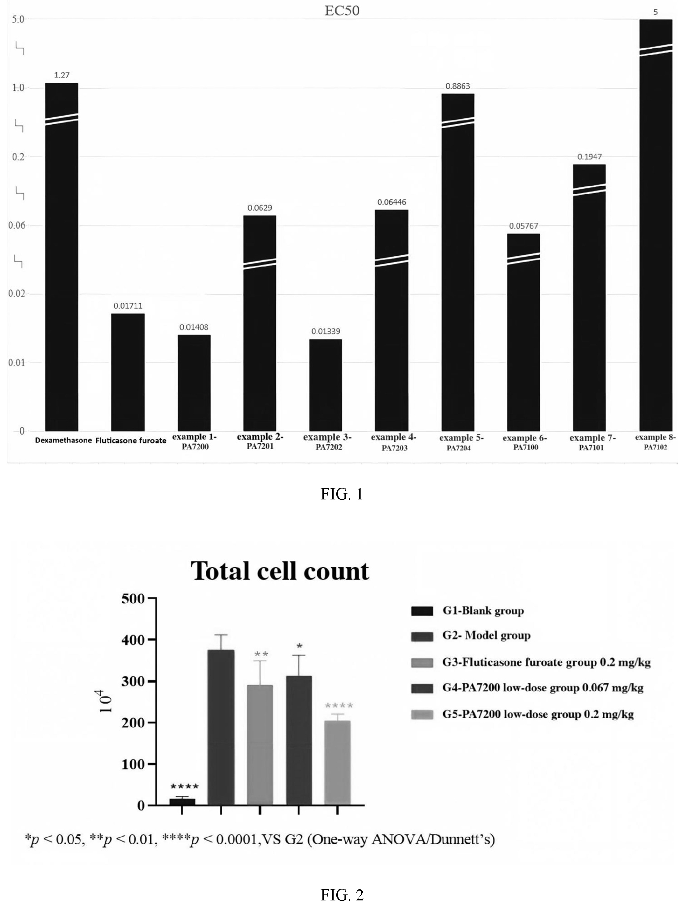

GRC9/HEK293 cells were cultured in a medium containing 88% phenol red-containing DMEM, 10% FBS, 1% P/S, 1% GlutaMax, 900 µg/mL G418 and 75 µg/mL hygromycin. Different candidate compounds (dexamethasone, fluticasone furoate, PA7200, PA7201, PA7202, PA7203, PA7204, PA7100, PA7101, PA7102) at various concentrations were added to each well of a 96-well plate. The cultured GRC9/HEK293 cells were then seeded into the 96-well plate using an inoculation medium containing 89% phenol red-free DMEM, 10% charcoal-stripped FBS and 1% GlutaMax. After incubation at 37 °C in a 5% CO₂ atmosphere for one day, the cells were harvested and luciferase activity was measured using a luciferase kit. The activity results are shown in Table 2 below and FIG. 1.

**Table 2: Summary of GR Reporter Gene Assay**

| No. | Sample ID | EC₅₀ (nM) |
|---|---|---|
| 1 | Dexamethasone | 1.270 |
| 2 | Fluticasone furoate | 0.01711 |
| 3 | Compound PA7200 | 0.01408 |
| 4 | Compound PA7201 | 0.0629 |
| 5 | Compound PA7202 | 0.01339 |
| 6 | Compound PA7203 | 0.06446 |
| 7 | Compound PA7204 | 0.8863 |
| 8 | Compound PA7100 | 0.05767 |
| 9 | Compound PA7101 | 0.1947 |
| 10 | Compound PA7102 | 5 |

### Result Analysis

The experimental results show that the EC₅₀ value of the commonly used glucocorticoid drug dexamethasone is 1.270 nM, indicating low activity in activating the glucocorticoid receptor; whereas the EC₅₀ values of the compounds PA7200 (Example 1) and PA7202 (Example 3) of the present application are 0.01408 nM and 0.01339 nM, respectively, which are far lower than that of dexamethasone, and also lower than that of the latest marketed glucocorticoid drug fluticasone furoate, representing a decrease of 18% and 22%, respectively, compared with fluticasone furoate (EC₅₀ = 0.01711 nM). This indicates that the glucocorticoid receptor activity of the compounds of the present application is significantly stronger than that of dexamethasone and fluticasone furoate, and possesses excellent activity in activating the glucocorticoid receptor.

### Example 10: Determination of Anti-inflammatory Effect

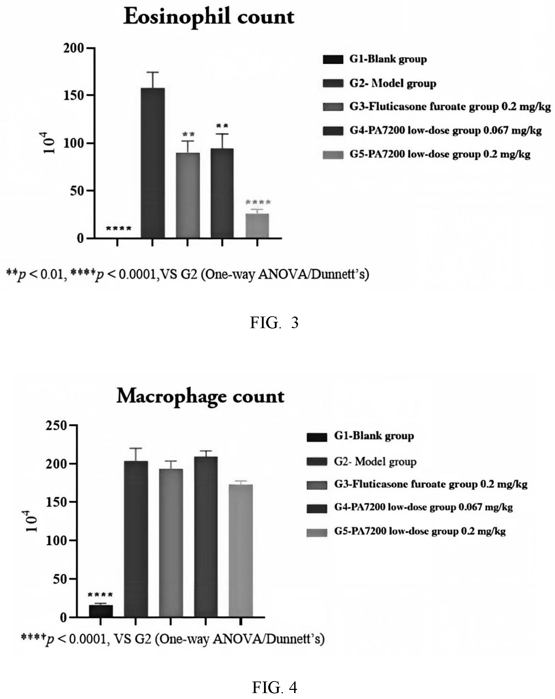

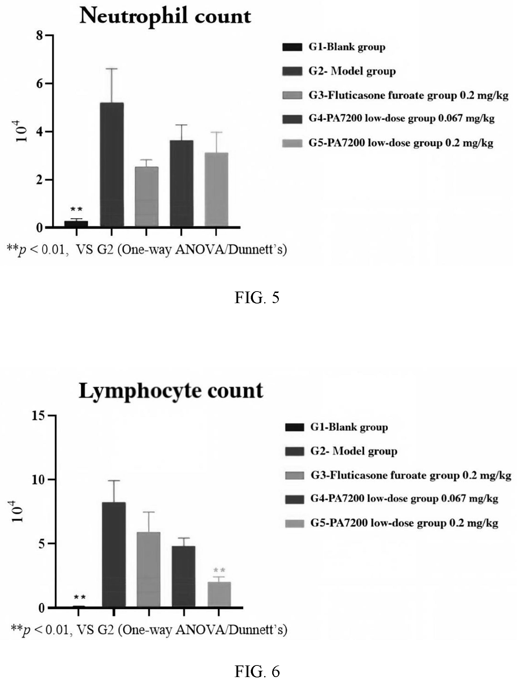

An OVA-induced mouse asthma model was used to evaluate the anti-inflammatory effect of the candidate compounds. First, 7-9 week-old Balb/c mice were immunized with OVA (ovalbumin). On day 1, the animals were randomly assigned to 5 different treatment groups (blank group, model group, positive drug group, test article low-dose group and test article high-dose group) using the BioBook random allocation function. On days 28-30 after the first immunization, the mice were challenged with OVA via the airway after nebulization using a BUXCO nebulized challenge solution, and different treatments were administered simultaneously, namely: blank group (normal saline), model group (no drug administered), positive drug group (0.2 mg/kg fluticasone furoate), test article low-dose group (0.067 mg/kg PA7200), and test article high-dose group (0.2 mg/kg PA7200). The lung tissues of the experimental animals in each group were lavaged and bronchoalveolar lavage fluid (BALF) was collected. Subsequently, the cells in the BALF were differentially counted and compared. The cell count results are shown in Table 3 below and FIGS. 2-6.

**Table 3: Cell Counts in BALF**

| Group | | Total cell count (×10⁴) | Eosinophil count (×10⁴) | Macrophage count (×10⁴) | Neutrophil count (×10⁴) | Lymphocyte count (×10⁴) |
|---|---|---|---|---|---|---|
| G1-Blank group | Mean | 16.59 | 0.17 | 16.01 | 0.28 | 0.14 |
| | SE | 2.49 | 0.02 | 2.39 | 0.09 | 0.02 |
| G2- Model group | Mean | 374.67 | 157.53 | 203.73 | 5.21 | 8.21 |
| | SE | 13.20 | 16.55 | 16.69 | 1.40 | 1.71 |
| G3-Fluticasone furoate group 0.2 mg/kg | Mean | 291.09 | 89.55 | 193.09 | 2.54 | 5.92 |
| | SE | 20.49 | 12.73 | 10.46 | 0.29 | 1.56 |
| G4-PA7200 low-dose group 0.067 mg/kg | Mean | 312.23 | 94.16 | 209.63 | 3.64 | 4.81 |
| | SE | 17.79 | 15.39 | 7.14 | 0.63 | 0.64 |
| G5-PA7200 | Mean | 203.53 | 25.34 | 173.04 | 3.12 | 2.03 |
| low-dose group 0.2 mg/kg | SE | 5.86 | 5.16 | 4.69 | 0.85 | 0.39 |

### Result Analysis

The experimental results show that, compared with the blank group, the total cell count and differential leukocyte counts in the BALF of the animals in the model group were significantly increased after OVA sensitization and challenge, presenting relatively typical pathological features of asthma, indicating that the asthma model was successfully established.

Compared with the model group, the PA7200 low-dose group showed significantly decreased total cell count and eosinophil count, and decreased neutrophil and lymphocyte counts to a certain extent; the PA7200 high-dose group showed significantly decreased total cell count, eosinophil count and lymphocyte count, and decreased neutrophil count to a certain extent, indicating that PA7200 has a good effect on ameliorating the inflammatory symptoms of asthma.

The fluticasone furoate group and the PA7200 high-dose group were administered at the same dose. However, compared with the fluticasone furoate group, the total cell count, eosinophil count and lymphocyte count in the PA7200 high-dose group were significantly lower, indicating that the anti-inflammatory effect of the compound PA7200 of the present application is significantly superior to that of the latest marketed glucocorticoid drug fluticasone furoate.

In summary, the compound PA7200 of the present application has an excellent effect in ameliorating the inflammatory symptoms of asthma, and is significantly superior to the latest marketed glucocorticoid drug fluticasone furoate.

It should be understood that, although the present invention has been described in conjunction with specific embodiments thereof, the foregoing description is intended to illustrate and not to limit the scope of the present invention, which is defined by the scope of the appended claims. Other aspects, advantages and modifications are within the scope of the claims.

## Claims

1. A compound represented by Formula I, or a pharmaceutically acceptable salt thereof, 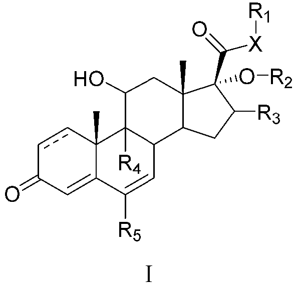
wherein, X is O or S;
-̅ -̅ -̅ is a single bond or a double bond;
R₁ is selected from the group consisting of hydrogen, C₁₋₆ alkyl, C₃₋₁₀ cycloalkyl, C₆₋₁₂ aryl, 5-to 15-membered heteroaryl, C₅₋₁₅ aralkyl, heteroaralkyl, and 3- to 10-membered heterocycloalkyl, wherein 0, 1, 2 or 3 hydrogen atoms in each of the foregoing groups are substituted with Rₐ;
R₂ is -L-R', wherein L is a bond or -C(O)-, and R' is selected from the group consisting of hydrogen, C₁₋₆ alkyl, C₃₋₁₀ cycloalkyl, C₆₋₁₂ aryl, 5- to 15-membered heteroaryl, C₅₋₁₅ aralkyl, heteroaralkyl, and 3- to 10-membered heterocycloalkyl, wherein 0, 1, 2 or 3 hydrogen atoms in the foregoing groups are substituted with R_{b};
R₃ is selected from the group consisting of hydrogen and C₁₋₆ alkyl, wherein 0, 1, 2 or 3 hydrogen atoms in the C₁₋₆ alkyl are substituted with R_{c};
R₄ and R₅ are each independently selected from the group consisting of hydrogen, halogen, and C₁₋₄ alkyl; wherein 0, 1, 2 or 3 hydrogen atoms in the C₁₋₄ alkyl are substituted with R_{d};
Rₐ, R_{b}, R_{c} and R_{d} are each independently selected from the group consisting of hydroxyl, halogen, CN, oxo, carboxyl, amino, sulfonic acid group, C₁₋₆ alkyl, and C₁₋₆ alkoxy.

2. The compound according to claim 1, **characterized in that**, R₁ is selected from the group consisting of hydrogen and C₁₋₆ alkyl; wherein 0, 1, 2 or 3 hydrogen atoms in the C₁₋₆ alkyl are substituted with halogen;
preferably, R₁ is selected from the group consisting of methyl, ethyl, propyl, isopropyl, butyl, isobutyl, and tert-butyl, wherein 0, 1, 2 or 3 hydrogen atoms in the foregoing groups are substituted with halogen;
more preferably, R₁ is fluoromethyl.

3. The compound according to claim 2, **characterized in that**, R₁ is selected from the group consisting of C₃₋₇ cycloalkyl, C₆₋₁₀ aryl, phenyl, and naphthyl, wherein 1, 2 or 3 hydrogen atoms in each of the foregoing groups are substituted with Rₐ.

4. The compound according to claim 1, **characterized in that**, R₂ is -C(O)-R', and R' is selected from the group consisting of hydrogen, C₁₋₆ alkyl, C₆₋₁₀ aryl(C₁₋₆ alkyl), 5- to 15-membered heteroaryl, and 5- to 15-membered heteroaryl(C₁₋₆ alkyl), wherein 0, 1, 2 or 3 hydrogen atoms in the foregoing groups are substituted with R_{b}, and R_{b} is selected from the group consisting of halogen, CN, carboxyl, and oxo; preferably, R₂ is selected from the group consisting of

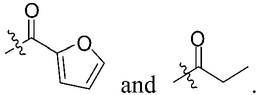

5. The compound according to claim 1, **characterized in that**, R₃ is selected from the group consisting of methyl, ethyl, propyl, isopropyl, butyl, isobutyl, and tert-butyl, wherein 1, 2 or 3 hydrogen atoms in the foregoing groups are substituted with R_{c}.

6. The compound according to claim 1, **characterized in that**, R₄ and R₅ are each independently -F.

7. The compound according to claim 1, **characterized in that**, the compound of Formula I is selected from the group consisting of:

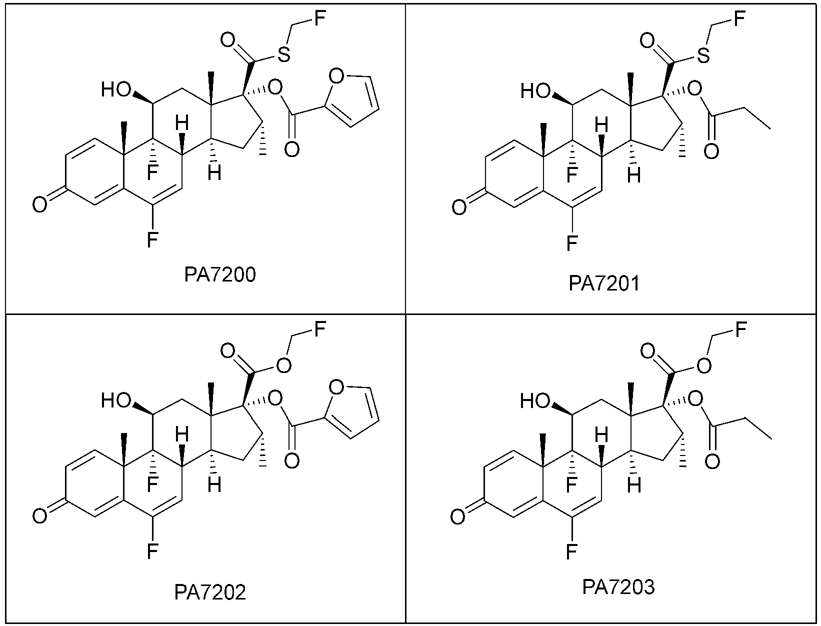

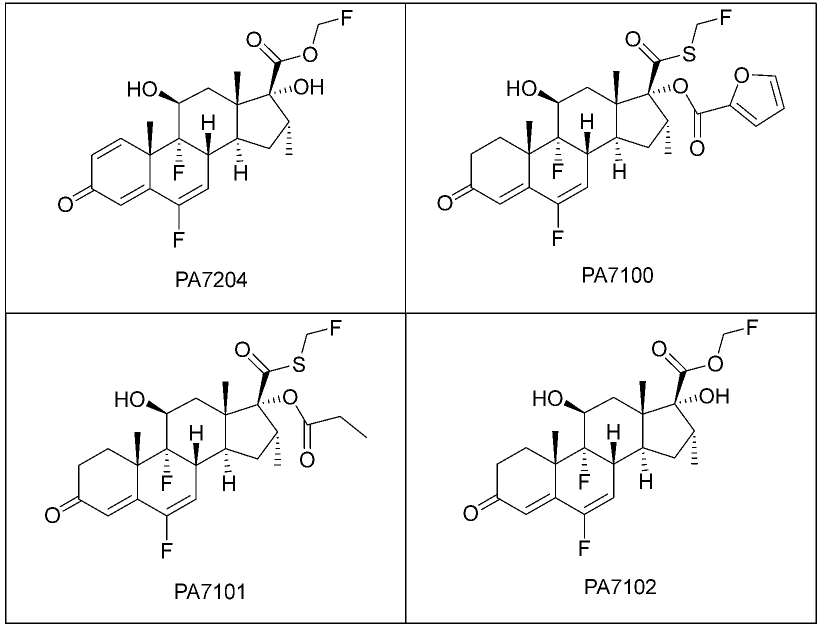

8. A pharmaceutical composition, **characterized in that**, the pharmaceutical composition comprises: (i) the compound according to any one of claims 1 to 7 or a pharmaceutically acceptable salt thereof; and (ii) a pharmaceutically acceptable carrier or adjuvant.

9. A method for modulating the activity of a glucocorticoid receptor in a biological sample, **characterized by** comprising the step of: contacting the glucocorticoid receptor in a biological sample with the compound according to any one of claims 1 to 7 or a pharmaceutically acceptable salt thereof, or the pharmaceutical composition according to claim 8.

10. Use of the compound according to any one of claims 1 to 7 or a pharmaceutically acceptable salt thereof, or the pharmaceutical composition according to claim 8, in the manufacture of a medicament for alleviating or treating an inflammatory disease;
preferably, the disease is selected from the group consisting of rhinitis, asthma, chronic obstructive pulmonary disease, dermatitis, arthritis, lupus, Crohn's disease, inflammatory bowel disease, celiac disease, glomerulonephritis, acne vulgaris, leukemia, and pancreatic cancer.
